(19) Europäisches Patentamt
European Patent Office
Office européen des brevets

(11) EP 2 272 344 A2

(12) **EUROPÄISCHE PATENTANMELDUNG**

(43) Veröffentlichungstag:
**12.01.2011 Patentblatt 2011/02**

(51) Int Cl.:
***A01N 43/40*** *(2006.01)* ***A01N 43/78*** *(2006.01)*

(21) Anmeldenummer: **10175682.3**

(22) Anmeldetag: **21.09.2005**

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **01.10.2004 DE 102004047922**

(62) Dokumentnummer(n) der früheren Anmeldung(en) nach Art. 76 EPÜ:
**05794725.1 / 1 796 462**

(71) Anmelder: **Bayer CropScience AG**
**40789 Monheim (DE)**

(72) Erfinder:
- **Jeschke, Peter, Dr.**
  **51467 Bergisch Gladbach (DE)**
- **Velten, Robert, Dr.**
  **40764 Langenfeld (DE)**
- **Lösel, Peter, Dr.**
  **51371 Leverkusen (DE)**
- **Malsam, Olga, Dr.**
  **51503 Rösrath (DE)**
- **Nauen, Ralf, Dr.**
  **40764 Langenfeld (DE)**
- **Görgens, Ulrich**
  **40882 Ratingen (DE)**
- **Tietjen, Klaus, Dr.**
  **40764 Langenfeld (DE)**
- **Arnold, Christian, Dr.**
  **40764 Langenfeld (DE)**
- **Hempel, Waltraud**
  **65835 Liederbach (DE)**
- **Sanwald, Erich, Dr.**
  **24159 Kiel (DE)**
- **Pitta, Leonardo**
  **51371 Leverkusen (DE)**

Bemerkungen:
Diese Anmeldung ist am 07-09-2010 als Teilanmeldung zu der unter INID-Code 62 erwähnten Anmeldung eingereicht worden.

(54) **Saatbehandlung**

(57) Die vorliegende Erfindung betrifft u.a. die Verwendung bekannter Verbindungen zur Behandlung von Saatgut.

EP 2 272 344 A2

## Beschreibung

**Wirkstoffe für die Saatgutbehandlung**

Die vorliegende Anmeldung betrifft die Verwendung bekannter Wirkstoffe für die Behandlung von Saatgut.
Sie betrifft weiterhin die Bekämpfung von tierischen Pflanzenschädlingen durch das Aufbringen bekannter Wirkstoffe auf den Boden.
Sie betrifft ferner die Verwendung bekannter Wirkstoffe zur Bekämpfung bestimmter Pflanzenschädlinge.
Die Wirkstoffe der Formel (I)

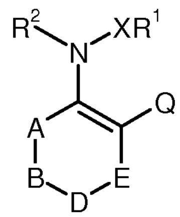

(I)

in welcher

$R^1$ für einen unsubstituierten oder substituierten 5- oder 6-gliedrigen aromatischen heterocyclischen Rest steht, der Stickstoff enthält,

X für jeweils unsubstituiertes oder substituiertes Alkylen oder Alkyliden steht,

$R^2$ für Wasserstoff, jeweils unsubstituiertes oder substituiertes Alkyl, Alkenyl, Cycloalkyl, Cycloalkenyl, Aryl oder für $YR^3$ steht,

Y für Sauerstoff, $S(O)_1$, CO oder $CO_2$ steht, 1 für 0, 1 oder 2 steht,

$R^3$ für Wasserstoff oder jeweils unsubstituiertes oder substituiertes Alkyl, Alkenyl, Alkinyl, Cycloalkyl, Cycloalkenyl oder Aryl steht,

A, B und D unabhängig voneinander jeweils für ein unsubstituiertes oder substituiertes Kohlenstoffatom oder Heteroatom oder für eine Einfachbindung stehen,

E für CO oder CS steht,

Q für Wasserstoff oder für jeweils unsubstituiertes oder substituiertes Alkyl, Alkenyl, Alkinyl oder Aryl oder für Nitro, Halogen oder für Z-$R^4$ steht,

Z für CO, $CO_2$ oder $S(O)_m$ steht,

m für 0, 1 oder 2 steht und

$R^4$ für jeweils unsubstituiertes oder substituiertes Alkyl, Alkenyl, Alkinyl, Cycloalkyl, Cycloalkenyl oder Aryl steht

und ihre Herstellung sind aus EP 0 539 588 A1 bekannt.
Im einzelnen sind in dieser Publikation die folgenden Verbindungen genannt:

**Tabelle 1**

| Verbindung Nr. | 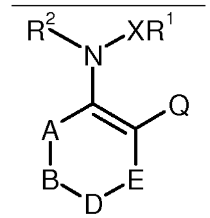 | | | | | Physikalische Eigenschaften [ ] Schmpkt. ˚C LC-MS; $^1$H-NMR (Lösungsmittel) vinyl-H [ppm] |
|---|---|---|---|---|---|---|
| | $R_1$-X | $R_2$ | -A-B-D | E | Q | |
| 1 | 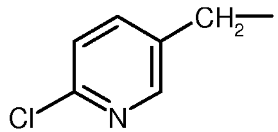 | H | $-CH_2-CH_2-CH_2-$ | 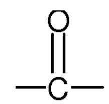 | H | [164-166] |
| 2 | 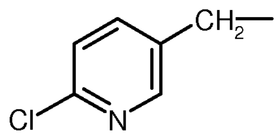 | $CH_3$ | $-CH_2-CH_2-CH_2-$ | 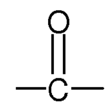 | H | [86-88] |
| 3 | 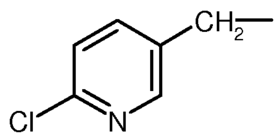 | $C_2H_5$ | $-CH_2-CH_2-CH_2-$ | 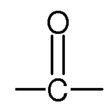 | H | $^1$H-NMR ($CDCl_3$): 5.22 |
| 4 | 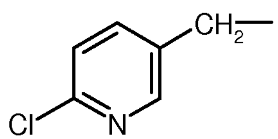 | n-$C_3H_7$ | $-CH_2-CH_2-CH_2-$ | 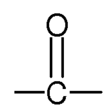 | H | $^1$H-NMR ($CDCl_3$): 5.20 |

(fortgesetzt)

| Verbindung Nr. | 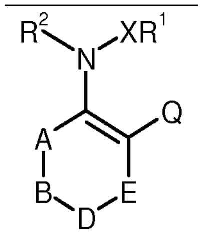 | | | | | Physikalische Eigenschaften [ ] Schmpkt. ˚C LC-MS; $^{1}$H-NMR (Lösungsmittel) vinyl-H [ppm] |
|---|---|---|---|---|---|---|
| | $R_1$-X | $R_2$ | -A-B-D | E | Q | |
| 5 | 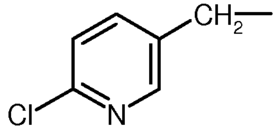 | i-$C_3H_7$ | $-CH_2-CH_2-CH_2-$ | 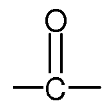 | H | |
| 6 | 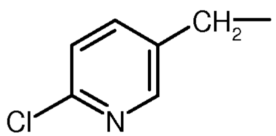 | 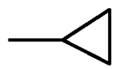 | $-CH_2-CH_2-CH_2-$ | 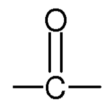 | H | |
| 7 | 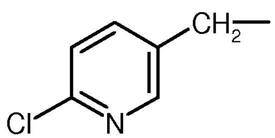 | 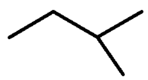 | $-CH_2-CH_2-CH_2-$ | 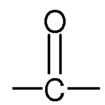 | H | $n_D^{25}$ 1.6005 |
| 8 | 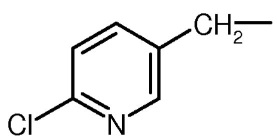 | 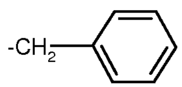 | $-CH_2-CH_2-CH_2-$ | 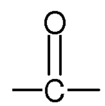 | H | |

(fortgesetzt)

| Verbindung Nr. | 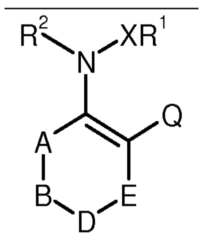 | | | | | Physikalische Eigenschaften [ ] Schmpkt. ˚C LC-MS; $^1$H-NMR (Lösungsmittel) vinyl-H [ppm] |
|---|---|---|---|---|---|---|
| | $R_1$-X | $R_2$ | -A-B-D | E | Q | |
| 9 | 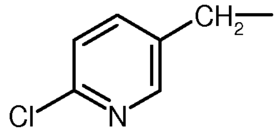 | $CH_2CH{=}CH_2$ | $-CH_2-CH_2-CH_2-$ | 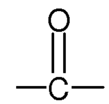 | H | $^1$H-NMR ($CDCl_3$): 5.26 |
| 10 | 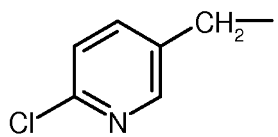 | $CH_2C{\equiv}CH$ | $-CH_2-CH_2-CH_2-$ | 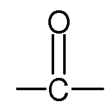 | H | $^1$H-NMR ($CDCl_3$): 5.34 |
| 11 | 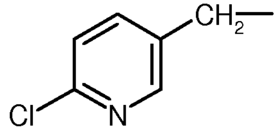 | $OCH_3$ | $-CH_2-CH_2-CH_2-$ | 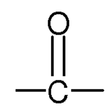 | H | [101-104] |
| 12 | 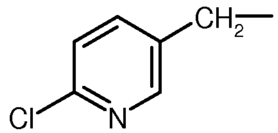 | $COCH_3$ | $-CH_2-CH_2-CH_2-$ | 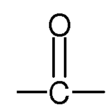 | H | |

(fortgesetzt)

| Verbindung Nr. | 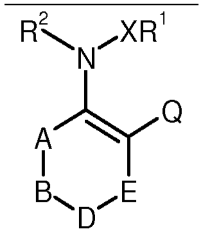 | | | | | Physikalische Eigenschaften [ ] Schmpkt. ˚C LC-MS; $^1$H-NMR (Lösungsmittel) vinyl-H [ppm] |
|---|---|---|---|---|---|---|
| | $R_1$-X | $R_2$ | -A-B-D | E | Q | |
| 13 | 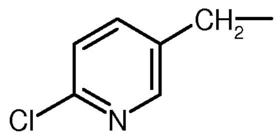 | $COCF_3$ | $-CH_2-CH_2-CH_2-$ | 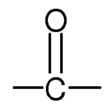 | H | $n_D^{25}$ 1.5303 |
| 14 | 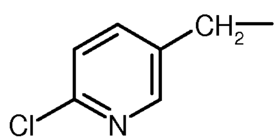 | 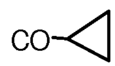 | $-CH_2-CH_2-CH_2-$ | 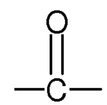 | H | |
| 15 | 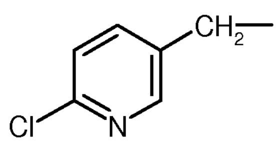 | $SO_2CH_3$ | $-CH_2-CH_2-CH_2-$ | 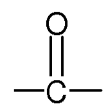 | H | |
| 16 | 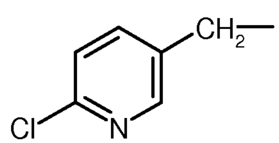 | 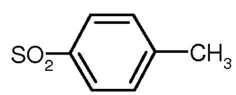 | $-CH_2-CH_2-CH_2-$ | 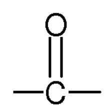 | H | |

(fortgesetzt)

| Verbindung Nr. | 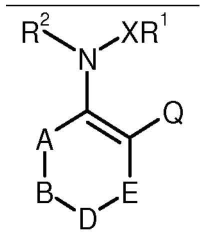 | | | | | Physikalische Eigenschaften [ ] Schmpkt. ˚C LC-MS; $^1$H-NMR (Lösungsmittel) vinyl-H [ppm] |
|---|---|---|---|---|---|---|
| | $R_1$-X | $R_2$ | -A-B-D | E | Q | |
| 17 | 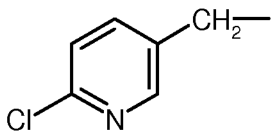 | H | $-CH_2-CH_2-CH_2-$ | 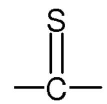 | H | [130-134] Zers. |
| 18 | 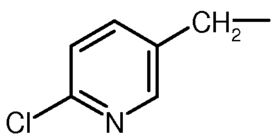 | $CH_3$ | $-CH_2-CH_2-CH_2-$ | 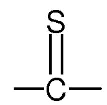 | H | |
| 19 | 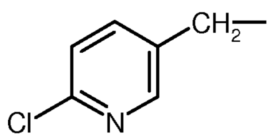 | H | $-CH_2-CH_2-CH_2-$ | 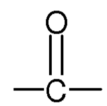 | $CH_3$ | [157-158] |
| 20 | 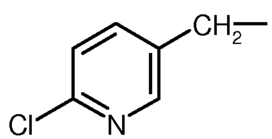 | H | $-CH_2-CH_2-CH_2-$ | 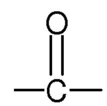 | $CH_2CH=CH_2$ | |

(fortgesetzt)

| Verbindung Nr. | 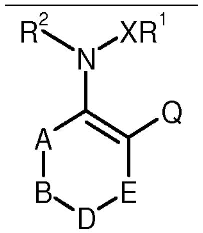 | | | | | Physikalische Eigenschaften [ ] Schmpkt. ˚C LC-MS; $^1$H-NMR (Lösungsmittel) vinyl-H [ppm] |
|---|---|---|---|---|---|---|
| | $R_1$-X | $R_2$ | -A-B-D | E | Q | |
| 21 | 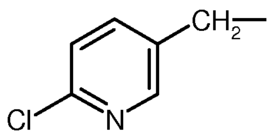 | H | $-CH_2CH_2CH_2-$ | 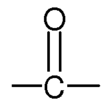 | $CH_2C{\equiv}CH$ | |
| 22 | 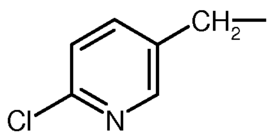 | H | $-CH_2CH_2CH_2-$ | 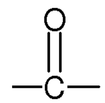 | 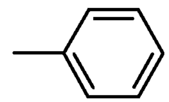 | |
| 23 | 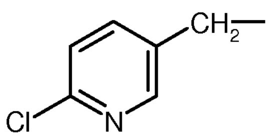 | H | $-CH_2CH_2CH_2-$ | 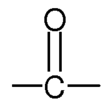 | Cl | [160-161] |
| 24 | 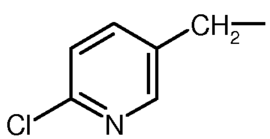 | H | $-CH_2CH_2CH_2-$ | 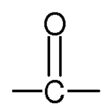 | $NO_2$ | $n_D^{25}$ 1.5908 |

(fortgesetzt)

| Verbindung Nr. | 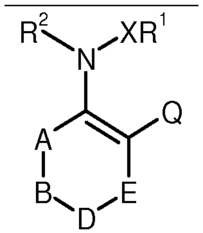 | | | | | Physikalische Eigenschaften [ ] Schmpkt. ˚C LC-MS; $^1$H-NMR (Lösungsmittel) vinyl-H [ppm] |
|---|---|---|---|---|---|---|
| | $R_1$-X | $R_2$ | -A-B-D | E | Q | |
| 25 | 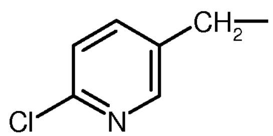 | H | $-CH_2CH_2CH_2-$ | 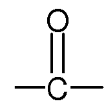 | CN | |
| 26 | 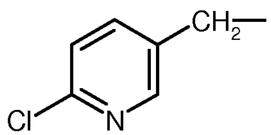 | H | $-CH_2CH_2CH_2-$ | 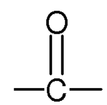 | $COCH_3$ | |
| 27 | 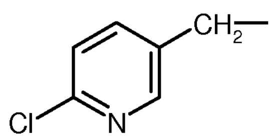 | H | $-CH_2CH_2CH_2-$ | 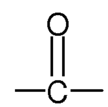 | $COCF_3$ | $n_D^{25}$ 1.5225 |
| 28 | 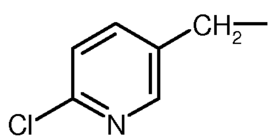 | H | $-CH_2CH_2CH_2-$ | 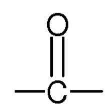 | $SO_2CH_3$ | |

(fortgesetzt)

| Verbindung Nr. | 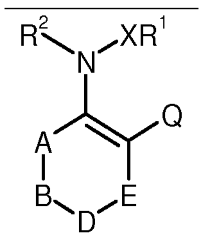 | | | | | Physikalische Eigenschaften [ ] Schmpkt. ˚C LC-MS; $^1$H-NMR (Lösungsmittel) vinyl-H [ppm] |
|---|---|---|---|---|---|---|
| | $R_1$-X | $R_2$ | -A-B-D | E | Q | |
| 29 | 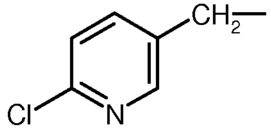 | H | 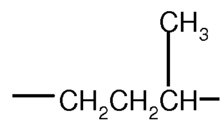 | 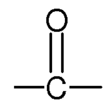 | H | [200-201] |
| 30 | 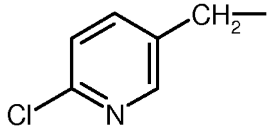 | H | 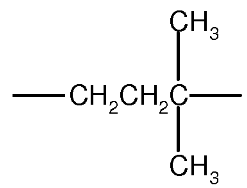 | 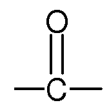 | H | [193-195] |
| 31 | 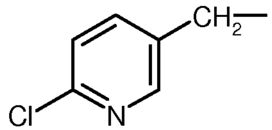 | H | 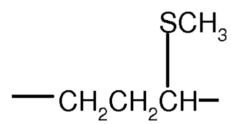 | 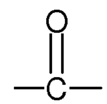 | H | [180-182] Zers. |
| 32 | 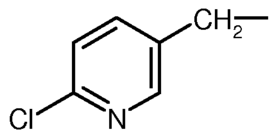 | H | 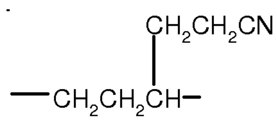 | 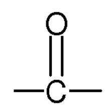 | H | [176-177] |

(fortgesetzt)

| Verbindung Nr. | 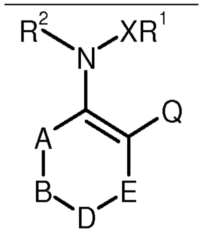 | | | | | Physikalische Eigenschaften [ ] Schmpkt. ˚C LC-MS; $^1$H-NMR (Lösungsmittel) vinyl-H [ppm] |
|---|---|---|---|---|---|---|
| | $R_1$-X | $R_2$ | -A-B-D | E | Q | |
| 33 | 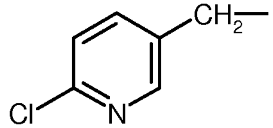 | H | 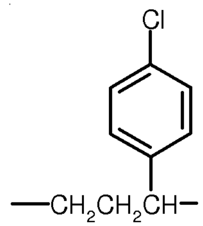 | 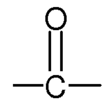 | H | [241-243] |
| 34 | 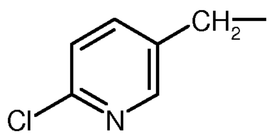 | H | 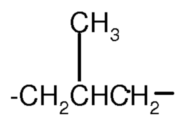 | 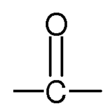 | H | [176-177] |
| 35 | 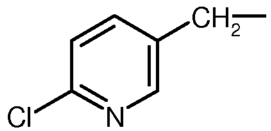 | H | 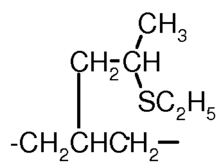 | 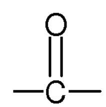 | H | [137-138] |

(fortgesetzt)

| Verbindung Nr. | 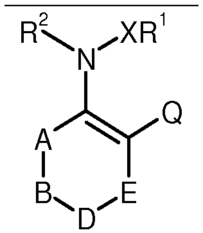 | | | | | Physikalische Eigenschaften [ ] Schmpkt. ˚C LC-MS; $^{1}$H-NMR (Lösungsmittel) vinyl-H [ppm] |
|---|---|---|---|---|---|---|
| | $R_1$-X | $R_2$ | -A-B-D | E | Q | |
| 36 | 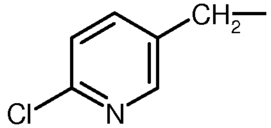 | H | 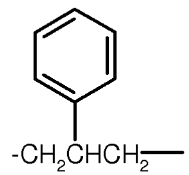 | 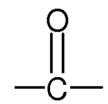 | H | [216-217] |
| 37 | 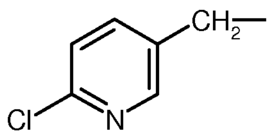 | H | 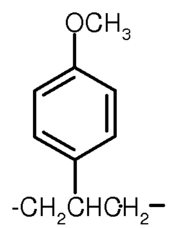 | 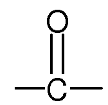 | H | [210-211] |
| 38 | 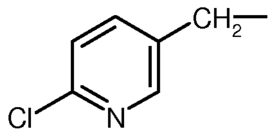 | H | 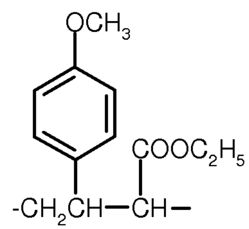 | 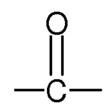 | H | [224-226] |

(fortgesetzt)

| Verbindung Nr. | 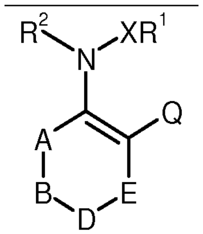 | | | | | Physikalische Eigenschaften [ ] Schmpkt. °C LC-MS; $^{1}$H-NMR (Lösungsmittel) vinyl-H [ppm] |
|---|---|---|---|---|---|---|
| | $R_1$-X | $R_2$ | -A-B-D | E | Q | |
| 39 | 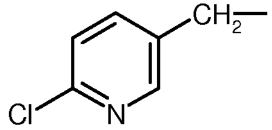 | H | 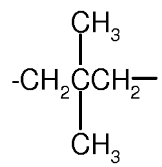 | 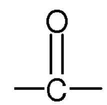 | H | [199-201] |
| 40 | 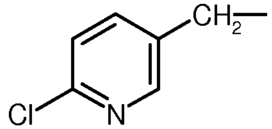 | H | 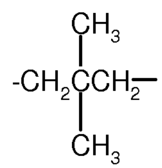 | 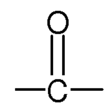 | $NO_2$ | [181-183] |
| 41 | 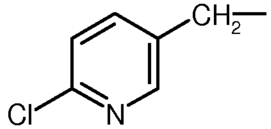 | H | 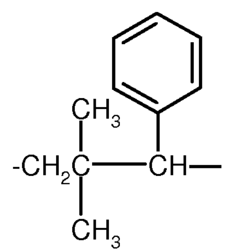 | 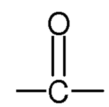 | H | [182-183] |
| 42 | 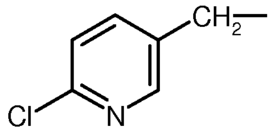 | $CH_3$ | 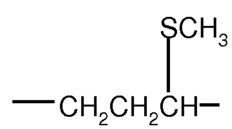 | 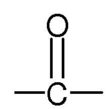 | H | [108-110] |

(fortgesetzt)

| Verbindung Nr. | 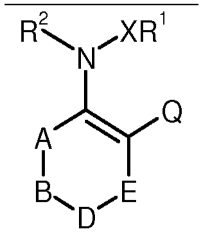 | | | | | Physikalische Eigenschaften [ ] Schmpkt. ˚C LC-MS; $^1$H-NMR (Lösungsmittel) vinyl-H [ppm] |
|---|---|---|---|---|---|---|
| | $R_1$-X | $R_2$ | -A-B-D | E | Q | |
| 43 | 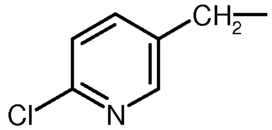 | $CH_3$ | 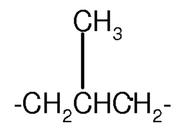 | 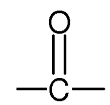 | H | 1.5992 |
| 44 | 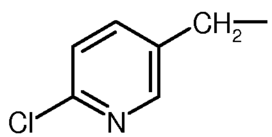 | H | 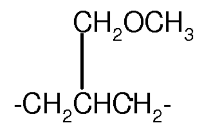 | 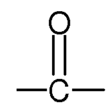 | H | [139-140] |
| 45 | 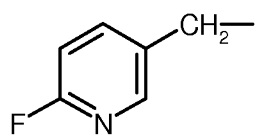 | H | $-CH_2CH_2CH_2-$ | 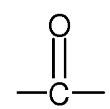 | H | |
| 46 | 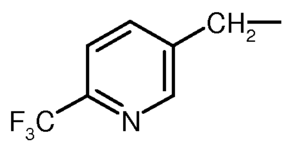 | H | $-CH_2CH_2CH_2-$ | 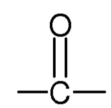 | H | |

(fortgesetzt)

| Verbindung Nr. | 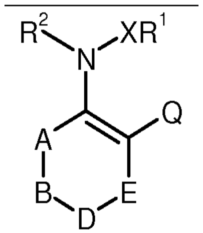 | | | | | Physikalische Eigenschaften [ ] Schmpkt. ˚C LC-MS; $^1$H-NMR (Lösungsmittel) vinyl-H [ppm] |
|---|---|---|---|---|---|---|
| | $R_1$-X | $R_2$ | -A-B-D | E | Q | |
| 47 | 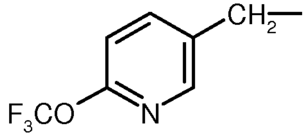 | H | $-CH_2CH_2CH_2-$ | 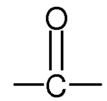 | H | |
| 48 | 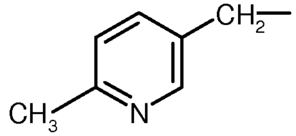 | H | $-CH_2CH_2CH_2-$ | 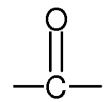 | H | |
| 49 | 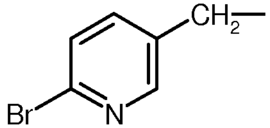 | H | $-CH_2CH_2CH_2-$ | 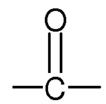 | H | |
| 50 | 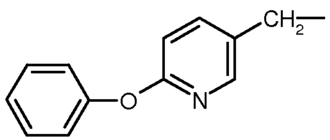 | H | $-CH_2CH_2CH_2-$ | 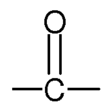 | H | |

(fortgesetzt)

| Verbindung Nr. | 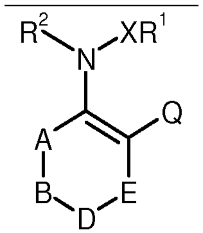 | | | | | Physikalische Eigenschaften [ ] Schmpkt. ˚C LC-MS; $^1$H-NMR (Lösungsmittel) vinyl-H [ppm] |
|---|---|---|---|---|---|---|
| | $R_1$-X | $R_2$ | -A-B-D | E | Q | |
| 51 | 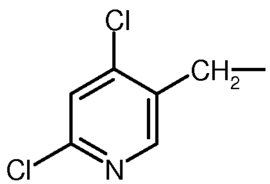 | H | $-CH_2CH_2CH_2-$ | 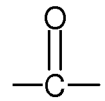 | H | |
| 52 | 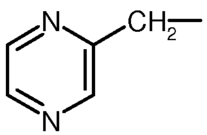 | H | $-CH_2CH_2CH_2-$ | 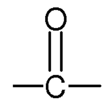 | H | |
| 53 | 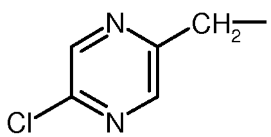 | H | $-CH_2CH_2CH_2-$ | 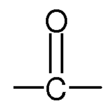 | H | |
| 54 | 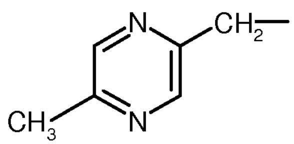 | H | $-CH_2CH_2CH_2-$ | 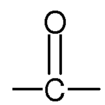 | H | |
| 55 | 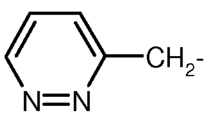 | H | $-CH_2CH_2CH_2-$ | 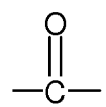 | H | |

(fortgesetzt)

| Verbindung Nr. | 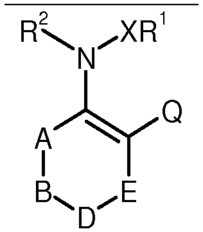 | | | | | Physikalische Eigenschaften [ ] Schmpkt. ˚C LC-MS; $^1$H-NMR (Lösungsmittel) vinyl-H [ppm] |
|---|---|---|---|---|---|---|
| | $R_1$-X | $R_2$ | -A-B-D | E | Q | |
| 56 | 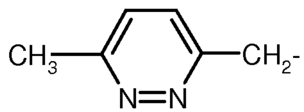 | H | $-CH_2CH_2CH_2-$ | 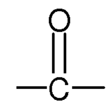 | H | |
| 57 | 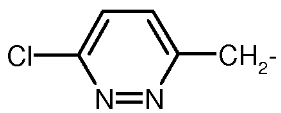 | H | $-CH_2CH_2CH_2-$ | 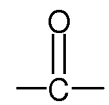 | H | |
| 58 | 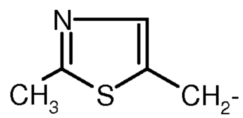 | H | $-CH_2CH_2CH_2-$ | 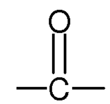 | H | |
| 59 | 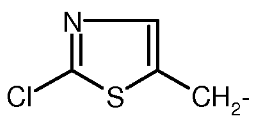 | H | $-CH_2CH_2CH_2-$ | 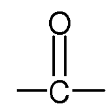 | H | [144-146] |
| 60 | 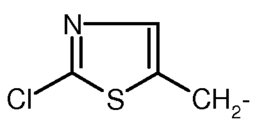 | $CH_3$ | $-CH_2CH_2CH_2-$ | 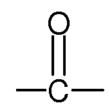 | H | $n_D^{25}$ 1.6208 |

(fortgesetzt)

| Verbindung Nr. | 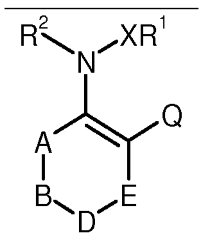 | | | | | Physikalische Eigenschaften [ ] Schmpkt. ˚C LC-MS; $^1$H-NMR (Lösungsmittel) vinyl-H [ppm] |
|---|---|---|---|---|---|---|
| | $R_1$-X | $R_2$ | -A-B-D | E | Q | |
| 61 | 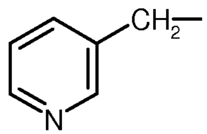 | H | $-CH_2CH_2CH_2-$ | 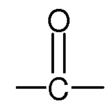 | H | [73-76] |
| 62 | 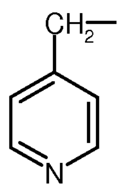 | H | $-CH_2CH_2CH_2-$ | 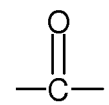 | H | |
| 63 | 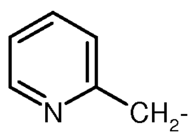 | H | $-CH_2CH_2CH_2-$ | 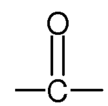 | H | |
| 64 | 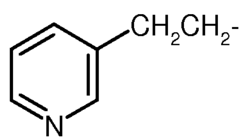 | H | $-CH_2CH_2CH_2-$ | 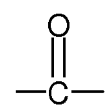 | H | |
| 65 | 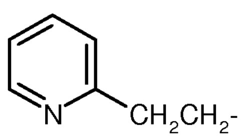 | H | $-CH_2CH_2CH_2-$ | 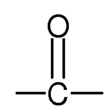 | H | |

(fortgesetzt)

| Verbindung Nr. | 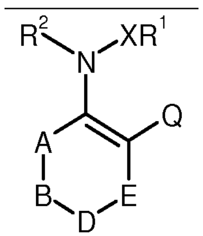 | | | | | Physikalische Eigenschaften [ ] Schmpkt. ˚C LC-MS; $^1$H-NMR (Lösungsmittel) vinyl-H [ppm] |
|---|---|---|---|---|---|---|
| | $R_1$-X | $R_2$ | -A-B-D | E | Q | |
| 66 | 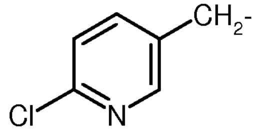 | H | $-CH_2CH_2NH-$ | 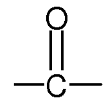 | H | [191-192] |
| 67 | 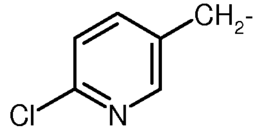 | H | 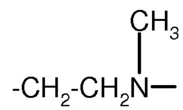 | 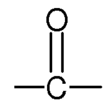 | H | |
| 68 | 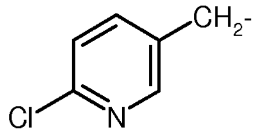 | H | 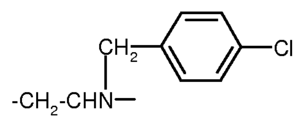 | 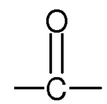 | H | [154-156] |
| 69 | 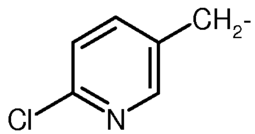 | H | 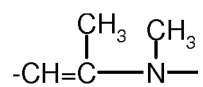 | 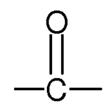 | $NO_2$ | $n_D^{25}$ 1.4972 |
| 70 | 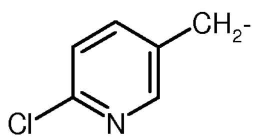 | H | 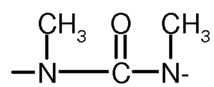 | 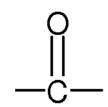 | H | [122-123] |

(fortgesetzt)

| Verbindung Nr. | 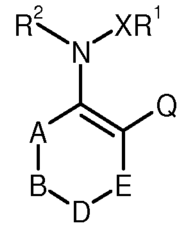 | | | | | Physikalische Eigenschaften [ ] Schmpkt. °C LC-MS; $^1$H-NMR (Lösungsmittel) vinyl-H [ppm] |
|---|---|---|---|---|---|---|
| | $R_1$-X | $R_2$ | -A-B-D | E | Q | |
| 71 | 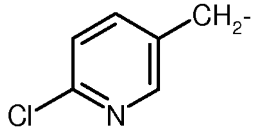 | H | 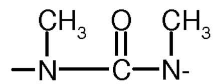 | 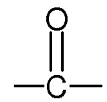 | $NO_2$ | [147-148] |
| 72 | 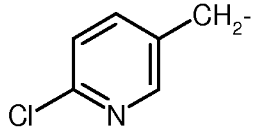 | H | 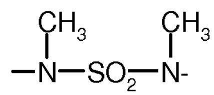 | 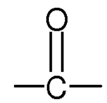 | H | [159-160] |
| 73 | 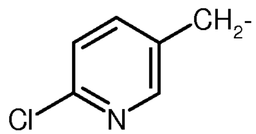 | H | 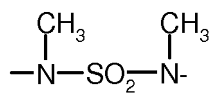 | 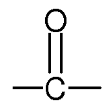 | $NO_2$ | [210-211] |
| 74 | 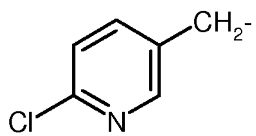 | H | $-CH_2-CH_2-O-$ | 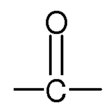 | H | [189-190] |
| 75 | 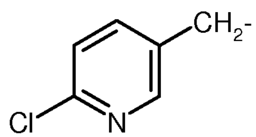 | H | 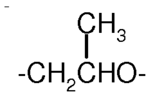 | 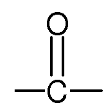 | H | [143-144] |

(fortgesetzt)

| Verbindung Nr. | 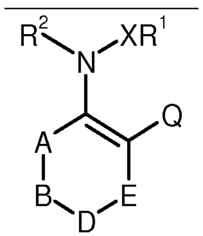 | | | | | Physikalische Eigenschaften [ ] Schmpkt. ˚C LC-MS; $^1$H-NMR (Lösungsmittel) vinyl-H [ppm] |
|---|---|---|---|---|---|---|
| | $R_1$-X | $R_2$ | -A-B-D | E | Q | |
| 76 | 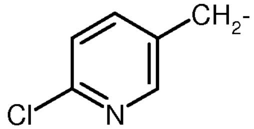 | H | 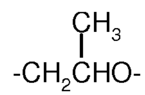 | 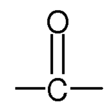 | $NO_2$ | [152-154] |
| 77 | 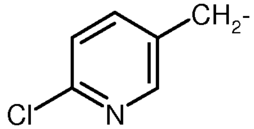 | H | 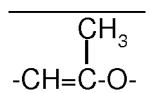 | 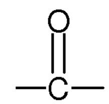 | H | [173-175] |
| 78 | 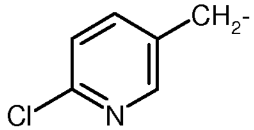 | H | 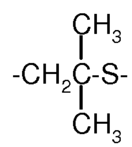 | 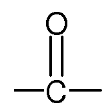 | H | [205-207] |
| 79 | 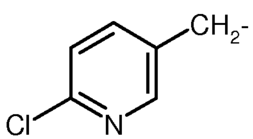 | H | 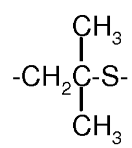 | 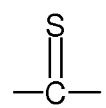 | H | [153-155] Zers. |
| 80 | 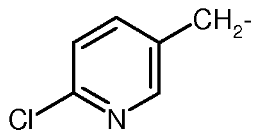 | H | $-CH_3SCH_2-$ | 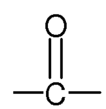 | H | [217-218] |

(fortgesetzt)

| Verbindung Nr. | 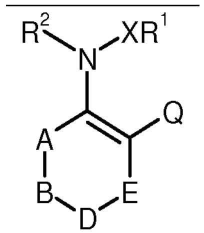 | | | | | Physikalische Eigenschaften [ ] Schmpkt. ˚C LC-MS; $^{1}$H-NMR (Lösungsmittel) vinyl-H [ppm] |
|---|---|---|---|---|---|---|
| | $R_1$-X | $R_2$ | -A-B-D | E | Q | |
| 81 | 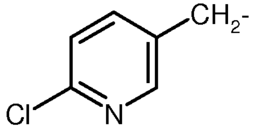 | H | 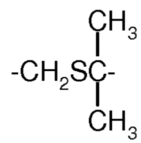 | 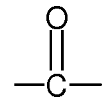 | H | [196-197] |
| 82 | 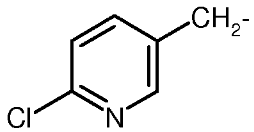 | $CH_3$ | $-CH_2CH_2O-$ | 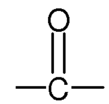 | H | [viskoses Öl] |
| 83 | 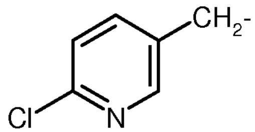 | H | $-CH_2O-$ | 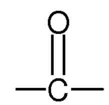 | H | [136-138] |
| 84 | 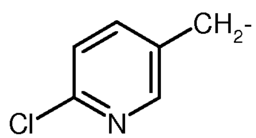 | $CH_3$ | $-CH_2O-$ | 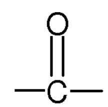 | H | [105-107] |
| 85 | 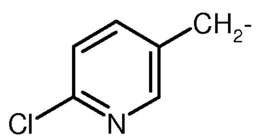 | $C_2H_5$ | $-CH_2O-$ | 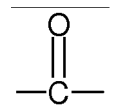 | H | [103-104] |

(fortgesetzt)

| Verbindung Nr. | 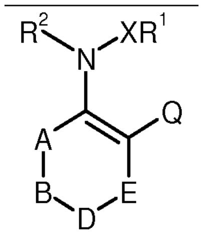 | | | | | Physikalische Eigenschaften [ ] Schmpkt. ˚C LC-MS; $^1$H-NMR (Lösungsmittel) vinyl-H [ppm] |
|---|---|---|---|---|---|---|
| | $R_1$-X | $R_2$ | -A-B-D | E | Q | |
| 86 | 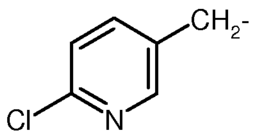 | n-$C_3H_7$ | -$CH_2$O- | 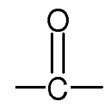 | H | [97-100] |
| 87 | 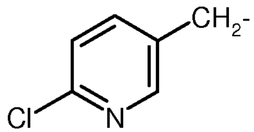 | i-$C_3H_7$ | -$CH_2$O- | 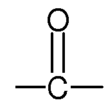 | H | $MH^+$ 267.1 (100); $^1$H-NMR ($CDCl_3$): 4.55 |
| 88 | 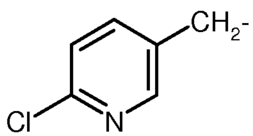 | 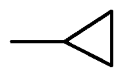 | -$CH_2$O- | 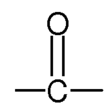 | H | [89-92] |
| 89 | 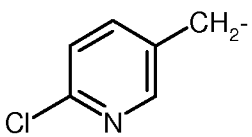 | 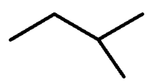 | -$CH_2$O- | 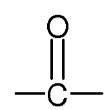 | H | $n_D^{25}$ 1.5725 |
| 90 | 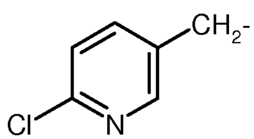 | 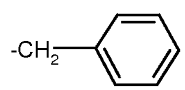 | -$CH_2$O- | 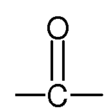 | H | $MH^+$ 315.1 (100); $^1$H-NMR (d3-Acetonitril): 4.70 |

(fortgesetzt)

| Verbindung Nr. | 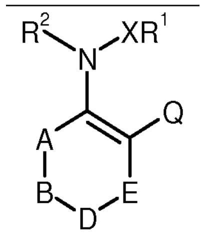 | | | | | Physikalische Eigenschaften [ ] Schmpkt. ˚C LC-MS; $^1$H-NMR (Lösungsmittel) vinyl-H [ppm] |
|---|---|---|---|---|---|---|
| | $R_1$-X | $R_2$ | -A-B-D | E | Q | |
| 91 | 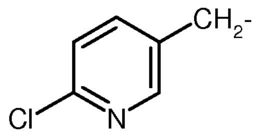 | $CH_2CH=CH_2$ | $-CH_2O-$ | 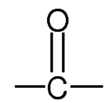 | H | [78-79] |
| 92 | 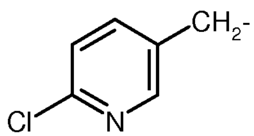 | $CH_2-C≡CH$ | $-CH_2O-$ | 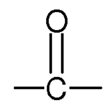 | H | $MH^+$ 263.0 (100); $^1$H-NMR (d3-Acetonitril) : 4.90 |
| 93 | 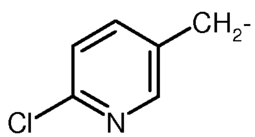 | $CONHCH_3$ | $-CH_2O-$ | 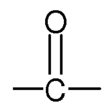 | H | [183-191] |
| 94 | 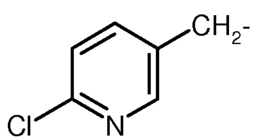 | $COCH_3$ | $-CH_2O-$ | 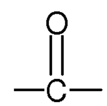 | H | $MH^+$ 267.0 (100); $^1$H-NMR ($CDCl_3$): 5.18 |
| 95 | 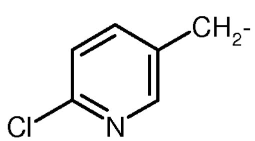 | $COCF_3$ | $-CH_2O-$ | 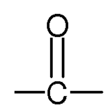 | H | |

(fortgesetzt)

| Verbindung Nr. | 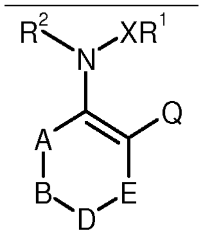 | | | | | Physikalische Eigenschaften [ ] Schmpkt. °C LC-MS; $^1$H-NMR (Lösungsmittel) vinyl-H [ppm] |
|---|---|---|---|---|---|---|
| | $R_1$-X | $R_2$ | -A-B-D | E | Q | |
| 96 | 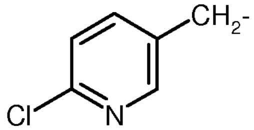 | 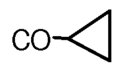 | $-CH_2O-$ | 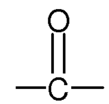 | H | [161-164] |
| 97 | 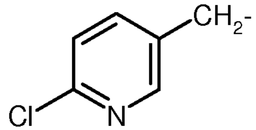 | $SO_2CH_3$ | $-CH_2O-$ | 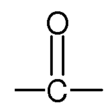 | H | |
| 98 | 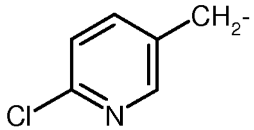 | $OCH_3$ | $-CH_2O-$ | 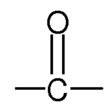 | H | [128-129] |
| 99 | 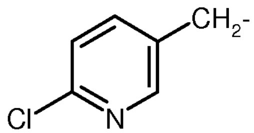 | H | $-CH_2O-$ | 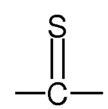 | H | |
| 100 | 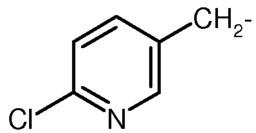 | $CH_3$ | $-CH_2O-$ | 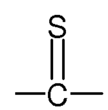 | H | |

(fortgesetzt)

| Verbindung Nr. | 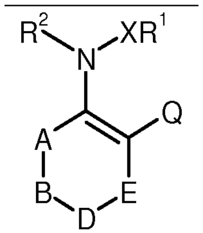 | | | | | Physikalische Eigenschaften [ ] Schmpkt. °C LC-MS; $^1$H-NMR (Lösungsmittel) vinyl-H [ppm] |
|---|---|---|---|---|---|---|
| | $R_1$-X | $R_2$ | -A-B-D | E | Q | |
| 101 | 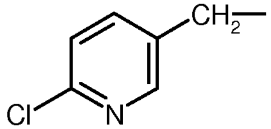 | H | $-CH_2O-$ | 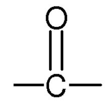 | $CH_3$ | |
| 102 | 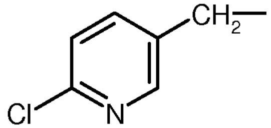 | H | $-CH_2O-$ | 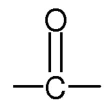 | Cl | |
| 103 | 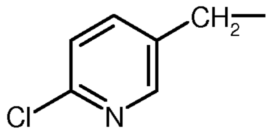 | H | $-CH_2O-$ | 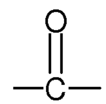 | Br | [144-145] |
| 104 | 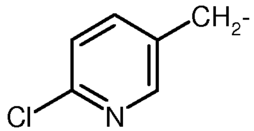 | $CH_3$ | $-CH_2O-$ | 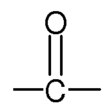 | F | [100-103] |
| 105 | 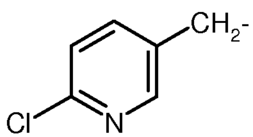 | 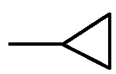 | $-CH_2O-$ | 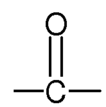 | F | [81-83] |

(fortgesetzt)

| Verbindung Nr. | 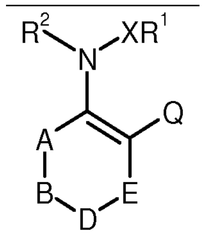 | | | | | Physikalische Eigenschaften [ ] Schmpkt. °C LC-MS; $^1$H-NMR (Lösungsmittel) vinyl-H [ppm] |
|---|---|---|---|---|---|---|
| | $R_1$-X | $R_2$ | -A-B-D | E | Q | |
| 106 | 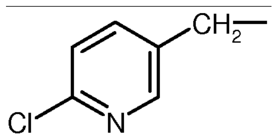 | H | $-CH_2O-$ | 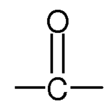 | $NO_2$ | [218-220] |
| 107 | 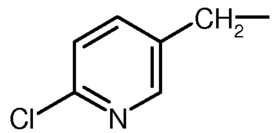 | H | $-CH_2O-$ | 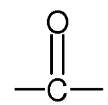 | CN | |
| 108 | 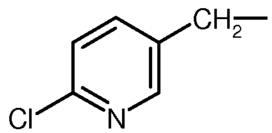 | H | $-CH_2O-$ | 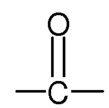 | $SO_2CH_3$ | [191-192] |
| 109 | 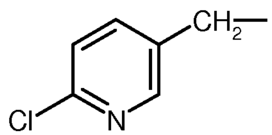 | H | $-CH_2O-$ | 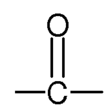 | $COH_3$ | |
| 110 | 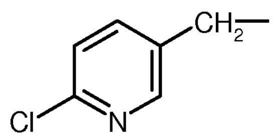 | H | $-CH_2O-$ | 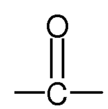 | $COCF_3$ | [173-176]Zers. |

(fortgesetzt)

| Verbindung Nr. | 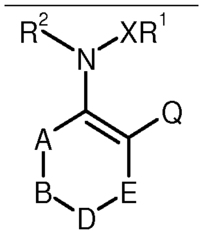 | | | | | Physikalische Eigenschaften [ ] Schmpkt. ˚C LC-MS; $^{1}$H-NMR (Lösungsmittel) vinyl-H [ppm] |
|---|---|---|---|---|---|---|
| | $R_1$-X | $R_2$ | -A-B-D | E | Q | |
| 111 | 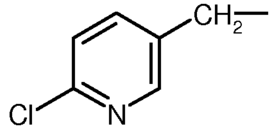 | H | $-CH_2S-$ | 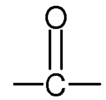 | H | [183-184] |
| 112 | 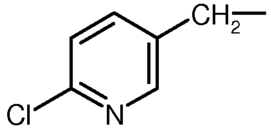 | $CH_3$ | $-CH_2S-$ | 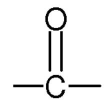 | H | [104-105] $MH^+$ 255 (100); Rt = 3,10* *Rt (in min) (0,1 % HCOOH/MeCN); vgl. WO 02085870 A |
| 113 | 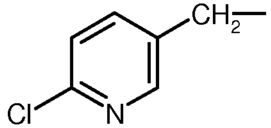 | $C_2H_5$ | $-CH_2S-$ | 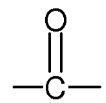 | H | $MH^+$ 269 (100); Rt= 3,61* *Rt (in min) (0,1 % HCOOH/ MeCN); vgl. WO 02085870 A |
| 114 | 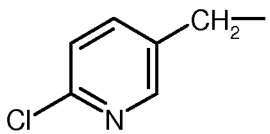 | 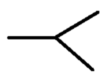 | $-CH_2S-$ | 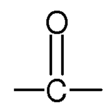 | H | |
| 115 | 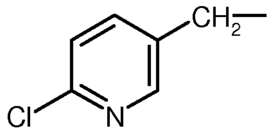 | 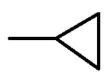 | $-CH_2S-$ | 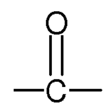 | H | |

(fortgesetzt)

| Verbindung Nr. | 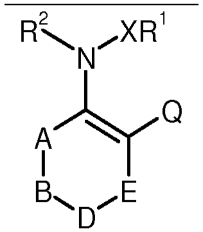 | | | | | Physikalische Eigenschaften [ ] Schmpkt. ˚C LC-MS; $^1H$-NMR (Lösungsmittel) vinyl-H [ppm] |
|---|---|---|---|---|---|---|
| | $R_1$-X | $R_2$ | -A-B-D | E | Q | |
| 116 | 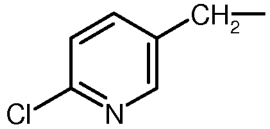 | H | 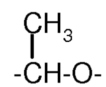 | 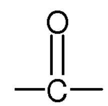 | H | [157-158] |
| 117 | 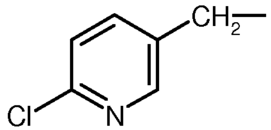 | $CH_3$ | 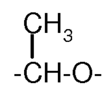 | 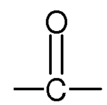 | H | $n_D^{25}$ 1.5737 |
| 118 | 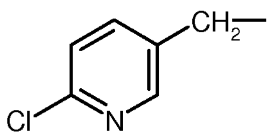 | $CH_3$ | 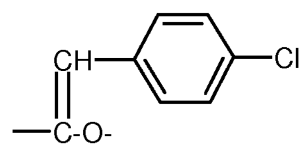 | 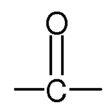 | H | [78-80] |
| 119 | 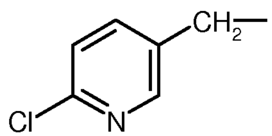 | $CH_3$ | 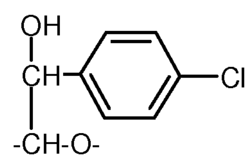 | 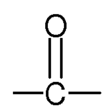 | H | [195-198] |
| 120 | 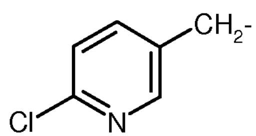 | $CH_3$ | 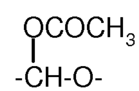 | 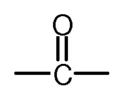 | H | [118-121] |

(fortgesetzt)

| Verbindung Nr. | 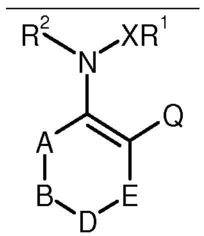 | | | | | Physikalische Eigenschaften [ ] Schmpkt. °C LC-MS; $^1$H-NMR (Lösungsmittel) vinyl-H [ppm] |
|---|---|---|---|---|---|---|
| | $R_1$-X | $R_2$ | -A-B-D | E | Q | |
| 121 | 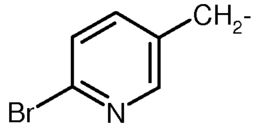 | $CH_3$ | $-CH_2O-$ | 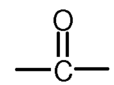 | H | $MH^+$ 283.0 (100); $^1$H-NMR (d3-Acetonitril): 4.64 |
| 122 | 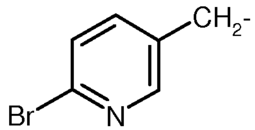 | 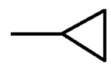 | $-CH_2O-$ | 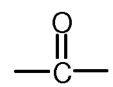 | H | |
| 123 | 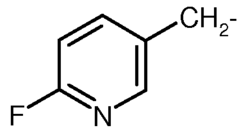 | $CH_3$ | $-CH_2O-$ | 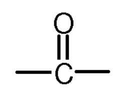 | H | $MH^+$ 223.1 (100); $^1$H-NMR (d3-Acetonitril): 4.75 |
| 124 | 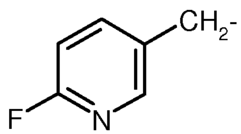 | 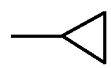 | $-CH_2O-$ | 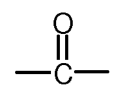 | H | $MH^+$ 249.1 (100); $^1$H-NMR (d3-Acetonitril): 4.75 |
| 125 | 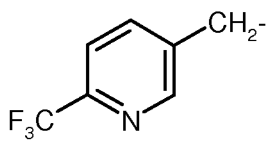 | $CH_3$ | $-CH_2O-$ | 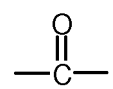 | H | $MH^+$ 273.1 (100); $^1$H-NMR (d3-Acetonitril): 4.64 |

(fortgesetzt)

| Verbindung Nr. | 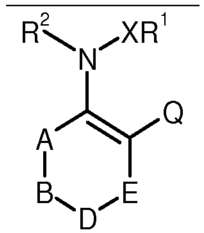 | | | | | Physikalische Eigenschaften [ ] Schmpkt. °C LC-MS; $^1$H-NMR (Lösungsmittel) vinyl-H [ppm] |
|---|---|---|---|---|---|---|
| | $R_1$-X | $R_2$ | -A-B-D | E | Q | |
| 126 | 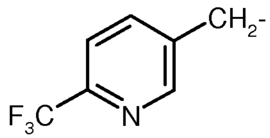 | 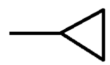 | -$CH_2$O- | 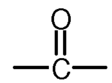 | H | $MH^+$ 299.1 (100); $^1$H-NMR (d3-Acetonitril): 4.72 |
| 127 | 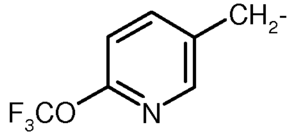 | $CH_3$ | -$CH_2$O- | 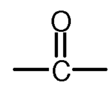 | H | |
| 128 | 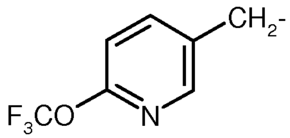 | 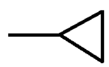 | -$CH_2$O- | 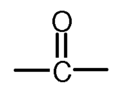 | H | |
| 129 | 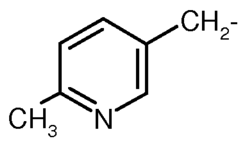 | $CH_3$ | -$CH_2$O- | 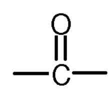 | H | |
| 130 | 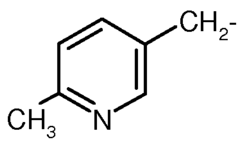 | 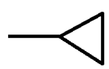 | -$CH_2$O- | 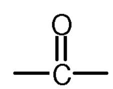 | H | |

(fortgesetzt)

| Verbindung Nr. | 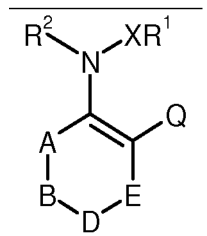 | | | | | Physikalische Eigenschaften [ ] Schmpkt. ˚C LC-MS; $^1$H-NMR (Lösungsmittel) vinyl-H [ppm] |
|---|---|---|---|---|---|---|
| | $R_1$-X | $R_2$ | -A-B-D | E | Q | |
| 131 | 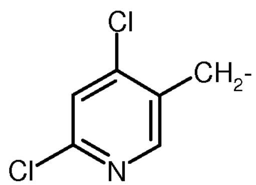 | $CH_3$ | $-CH_2O-$ | 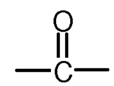 | H | |
| 132 | 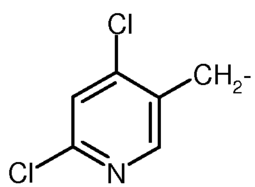 | 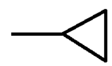 | $-CH_2O-$ | 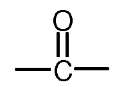 | H | |
| 133 | 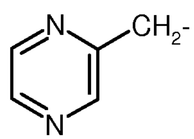 | $CH_3$ | $-CH_2O-$ | 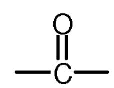 | H | |
| 134 | 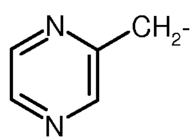 | 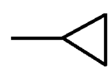 | $-CH_2O-$ | 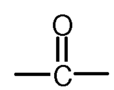 | H | |

(fortgesetzt)

| Verbindung Nr. | 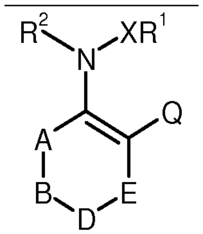 | | | | | Physikalische Eigenschaften [ ] Schmpkt. ˚C LC-MS; $^1$H-NMR (Lösungsmittel) vinyl-H [ppm] |
|---|---|---|---|---|---|---|
| | $R_1$-X | $R_2$ | -A-B-D | E | Q | |
| 135 | 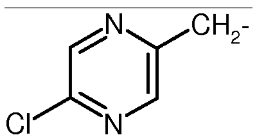 | $CH_3$ | $-CH_2O-$ | 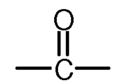 | H | |
| 136 | 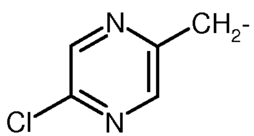 | 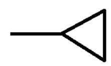 | $-CH_2O-$ | 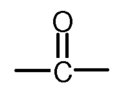 | H | |
| 137 | 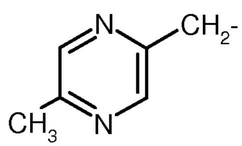 | $CH_3$ | $-CH_2O-$ | 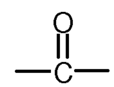 | H | |
| 138 | 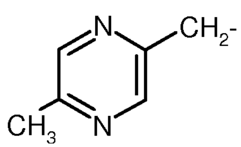 | 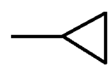 | $-CH_2O-$ | 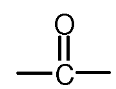 | H | |
| 139 | 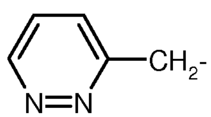 | $CH_3$ | $-CH_2O-$ | 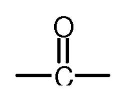 | H | |

(fortgesetzt)

| Verbindung Nr. | 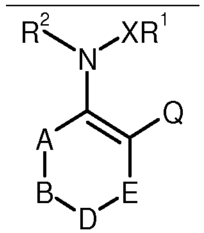 | | | | | Physikalische Eigenschaften [ ] Schmpkt. ˚C LC-MS; $^1$H-NMR (Lösungsmittel) vinyl-H [ppm] |
|---|---|---|---|---|---|---|
| | $R_1$-X | $R_2$ | -A-B-D | E | Q | |
| 140 | 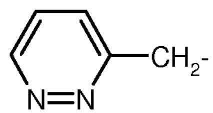 | 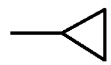 | $-CH_2O-$ | 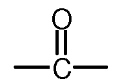 | H | |
| 141 | 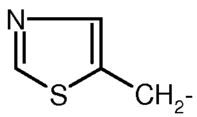 | $CH_3$ | $-CH_2O-$ | 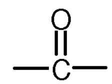 | H | |
| 142 | 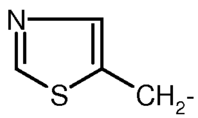 | 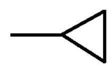 | $-CH_2O-$ | 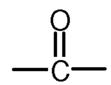 | H | |
| 143 | 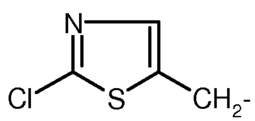 | H | $-CH_2O-$ | 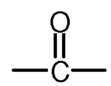 | H | [143-145] |
| 144 | 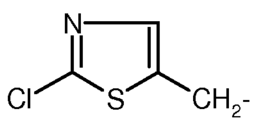 | $CH_3$ | $-CH_2O-$ | 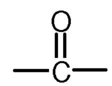 | H | $n_D^{25}$ 1.6035 |

(fortgesetzt)

| Verbindung Nr. | 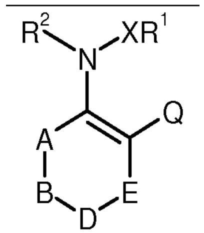 | | | | | Physikalische Eigenschaften [ ] Schmpkt. ˚C LC-MS; $^1$H-NMR (Lösungsmittel) vinyl-H [ppm] |
|---|---|---|---|---|---|---|
| | $R_1$-X | $R_2$ | -A-B-D | E | Q | |
| 145 | 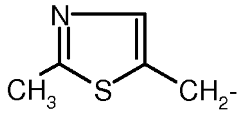 | $CH_3$ | -$CH_2$O- | 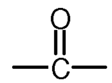 | H | |
| 146 | 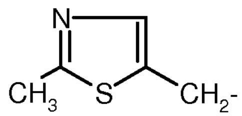 | 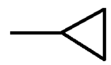 | -$CH_2$O- | 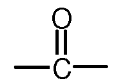 | H | |
| 147 | 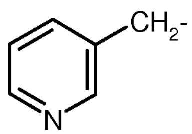 | $CH_3$ | -$CH_2$O- | 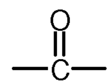 | H | $MH^+$ 205.1 (100); $^1$H-NMR (d3-Acetonitril): 4.63 |
| 148 | 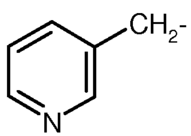 | 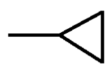 | -$CH_2$O- | 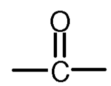 | H | |
| 149 | 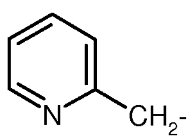 | $CH_3$ | -$CH_2$O- | 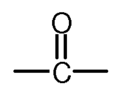 | H | |

(fortgesetzt)

| Verbindung Nr. | 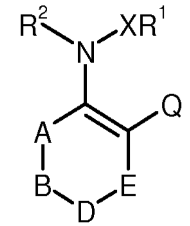 | | | | | Physikalische Eigenschaften [ ] Schmpkt. ˚C LC-MS; $^1$H-NMR (Lösungsmittel) vinyl-H [ppm] |
|---|---|---|---|---|---|---|
| | $R_1$-X | $R_2$ | -A-B-D | E | Q | |
| 150 | 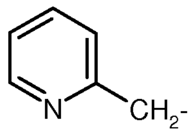 | 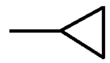 | $-CH_2O-$ | 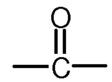 | H | |
| 151 | 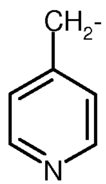 | $CH_3$ | $-CH_2O-$ | 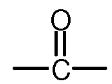 | H | |
| 152 | 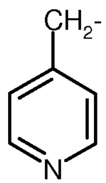 | 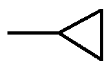 | $-CH_2O-$ | 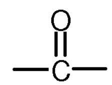 | H | |
| 153 | 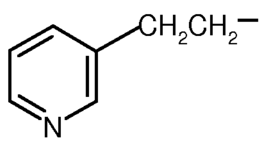 | $CH_3$ | $-CH_2O-$ | 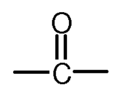 | H | |

(fortgesetzt)

| Verbindung Nr. | 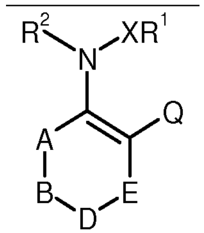 | | | | | Physikalische Eigenschaften [ ] Schmpkt. ˚C LC-MS; $^{1}$H-NMR (Lösungsmittel) vinyl-H [ppm] |
|---|---|---|---|---|---|---|
| | $R_1$-X | $R_2$ | -A-B-D | E | Q | |
| 154 | 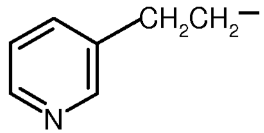 | 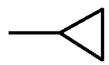 | -$CH_2$O- | 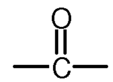 | H | |
| 155 | 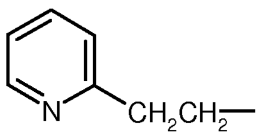 | $CH_3$ | -$CH_2$O- | 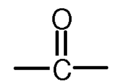 | H | |
| 156 | 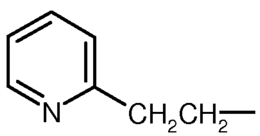 | 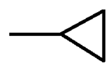 | -$CH_2$O- | 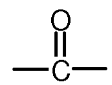 | H | |
| 157 | 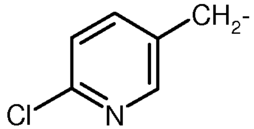 | H | -$CH_2$NH- | 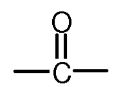 | H | [194-200] Zers. |
| 158 | 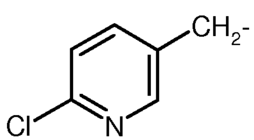 | $CH_3$ | -$CH_2$NH- | 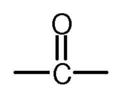 | H | [193-198] Zers. |

(fortgesetzt)

| Verbindung Nr. | 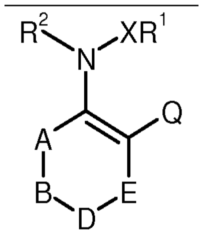 | | | | | Physikalische Eigenschaften [ ] Schmpkt. ˚C LC-MS; $^1$H-NMR (Lösungsmittel) vinyl-H [ppm] |
|---|---|---|---|---|---|---|
| | $R_1$-X | $R_2$ | -A-B-D | E | Q | |
| 159 | 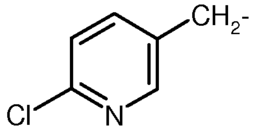 | H | 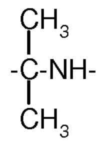 | 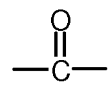 | H | [214-215] |
| 160 | 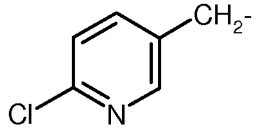 | $CH_3$ | 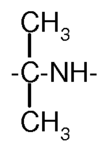 | 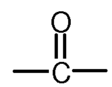 | H | [147-148] |
| 161 | 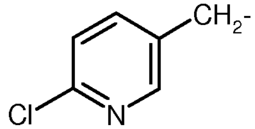 | H | 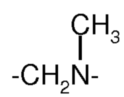 | 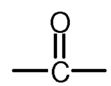 | H | [192-193] Zers. |
| 162 | 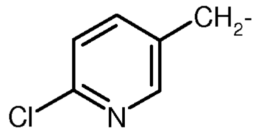 | $CH_3$ | 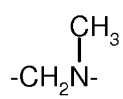 | 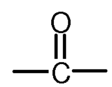 | H | [114-115] |
| 163 | 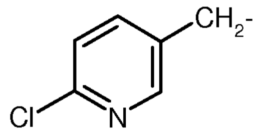 | H | 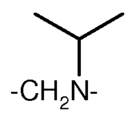 | 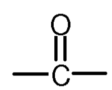 | H | [223-226] Zers. |

(fortgesetzt)

| Verbindung Nr. | 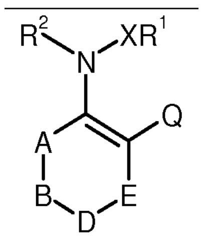 | | | | | Physikalische Eigenschaften [ ] Schmpkt. ˚C LC-MS; $^1$H-NMR (Lösungsmittel) vinyl-H [ppm] |
|---|---|---|---|---|---|---|
| | $R_1$-X | $R_2$ | -A-B-D | E | Q | |
| 164 | 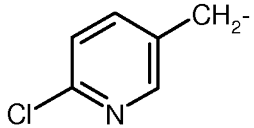 | $CH_3$ | 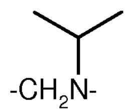 | 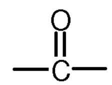 | H | [101-102] |
| 165 | 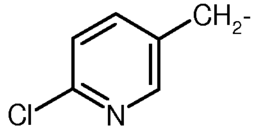 | $CH_3$ | 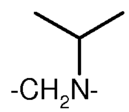 | 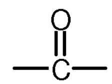 | 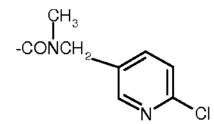 | [133-136] |
| 166 | 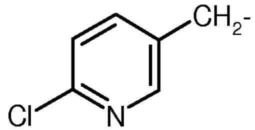 | n-$C_3H_7$ | $-CH_2NH-$ | 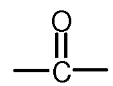 | H | |
| 167 | 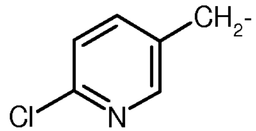 | i-$C_3H_7$ | $-CH_2NH-$ | 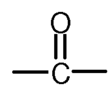 | H | |
| 168 | 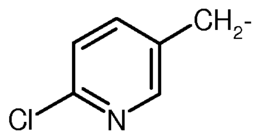 | 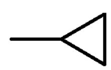 | $-CH_2NH-$ | 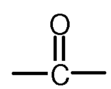 | H | $MH^+$ 264.0 (100); $^1$H-NMR (d6-DMSO): 4.77 |

(fortgesetzt)

| Verbindung Nr. | 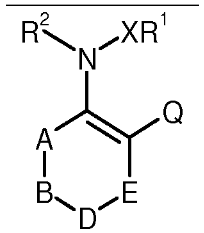 | | | | | Physikalische Eigenschaften [ ] Schmpkt. ˚C LC-MS; $^{1}$H-NMR (Lösungsmittel) vinyl-H [ppm] |
|---|---|---|---|---|---|---|
| | $R_1$-X | $R_2$ | -A-B-D | E | Q | |
| 169 | 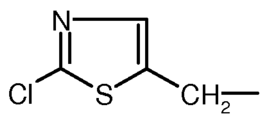 | $CH_3$ | $-CH_2NH-$ | 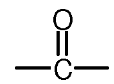 | H | $MH^+$ 244.0 (100); $^{1}$H-NMR (d6-DMSO): 4.80 |
| 170 | 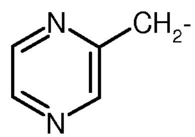 | $CH_3$ | $-CH_2NH-$ | 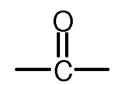 | H | |
| 171 | 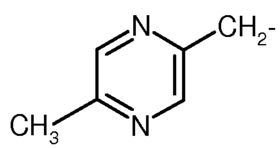 | $CH_3$ | $-CH_2NH-$ | 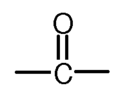 | H | |
| 172 | 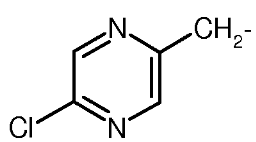 | $CH_3$ | $-CH_2NH-$ | 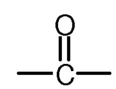 | H | |
| 173 | 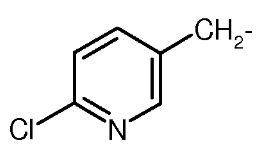 | $CH_3$ | $-CH_2NH-$ | 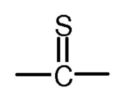 | H | |

(fortgesetzt)

| Verbindung Nr. | 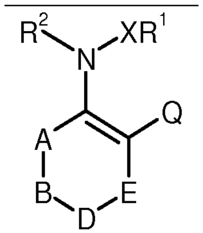 | | | | | Physikalische Eigenschaften [ ] Schmpkt. ˚C LC-MS; ¹H-NMR (Lösungsmittel) vinyl-H [ppm] |
|---|---|---|---|---|---|---|
| | $R_1$-X | $R_2$ | -A-B-D | E | Q | |
| 174 | 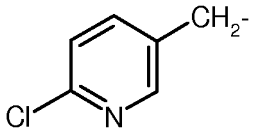 | H | $-CH_2CH_2-$ | 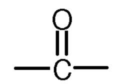 | H | [173-175] |
| 175 | 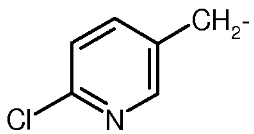 | $CH_3$ | $-CH_2CH_2-$ | 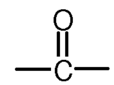 | H | $n_D^{25}$ 1.6092 |
| 176 | 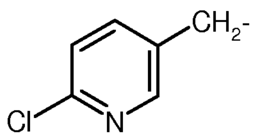 | $C_2H_5$ | $-CH_2CH_2-$ | 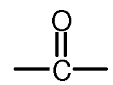 | H | |
| 177 | 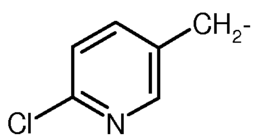 | $n-C_3H_7$ | $-CH_2CH_2-$ | 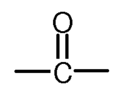 | H | |
| 178 | 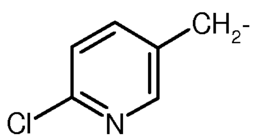 | $i-C_3H_7$ | $-CH_2CH_2-$ | 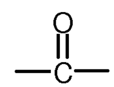 | H | |

(fortgesetzt)

| Verbindung Nr. | 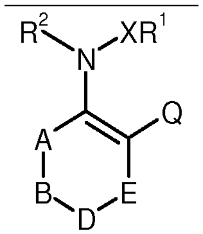 | | | | | Physikalische Eigenschaften [ ] Schmpkt. °C LC-MS; $^1$H-NMR (Lösungsmittel) vinyl-H [ppm] |
|---|---|---|---|---|---|---|
| | $R_1$-X | $R_2$ | -A-B-D | E | Q | |
| 179 | 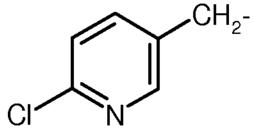 | 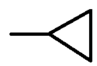 | $-CH_2CH_2-$ | 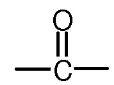 | H | $MH^+$ 263.1 (100); $^1$H-NMR (d6-DMSO): 5.05 |
| 180 | 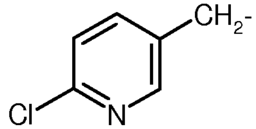 | $COCH_3$ | $-CH_2CH_2-$ | 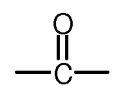 | H | |
| 181 | 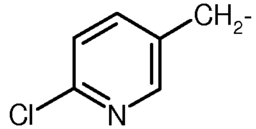 | $SO_2CH_3$ | $-CH_2CH_2-$ | 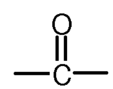 | H | |
| 182 | 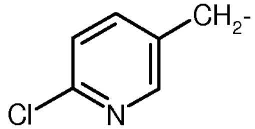 | $CH_3$ | $-CH_2CH_2-$ | 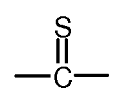 | H | |
| 183 | 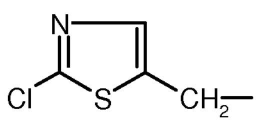 | $CH_3$ | $-CH_2CH_2-$ | 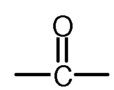 | H | |

(fortgesetzt)

| Verbindung Nr. | 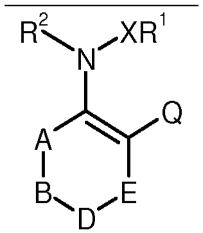 | | | | | Physikalische Eigenschaften [ ] Schmpkt. ˚C LC-MS; $^1$H-NMR (Lösungsmittel) vinyl-H [ppm] |
|---|---|---|---|---|---|---|
| | $R_1$-X | $R_2$ | -A-B-D | E | Q | |
| 184 | 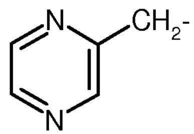 | $CH_3$ | $-CH_2CH_2-$ | 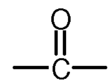 | H | |
| 185 | 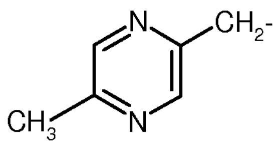 | $CH_3$ | $-CH_2CH_2-$ | 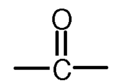 | H | |
| 186 | 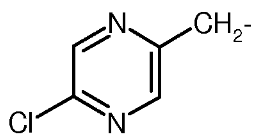 | $CH_3$ | $-CH_2CH_2-$ | 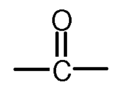 | H | |
| 187 | 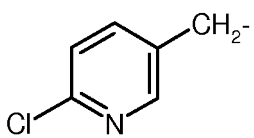 | $CH_3$ | $-CH_2CH_2-$ | 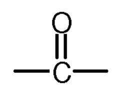 | $CH_3$ | [174-175] |
| 188 | 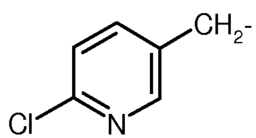 | H | $-CH_2CH_2-$ | 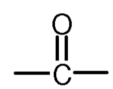 | $NO_2$ | [165-167] |

Ferner ist aus EP 0 539 588 A1 bekannt, dass die Verbindungen der Formel (I) eine gute Wirksamkeit gegen Heerwurm, Kohlmotte, Blattläuse, Zikaden und die braune Reiszikade aufweisen.

Jetzt wurde gefunden, dass die bekannten Verbindungen der Formel (I) sich hervorragend für die Behandlung von Saatgut eignen.

So entsteht ein großer Teil des durch Schädlinge verursachten Schadens an Kulturpflanzen bereits durch den Befall des Saatguts während der Lagerung und nach dem Einbringen des Saatguts in den Boden sowie während und unmittelbar nach der Keimung der Pflanzen. Diese Phase ist besonders kritisch, da die Wurzeln und Sprosse der wachsenden Pflanze besonders empfindlich sind und bereits ein geringer Schaden zum Absterben der ganzen Pflanze führen kann. Es besteht daher ein insbesondere großes Interesse daran, das Saatgut und die keimende Pflanze durch den Einsatz geeigneter Mittel zu schützen.

Die Bekämpfung von Schädlingen durch die Behandlung des Saatguts von Pflanzen ist seit langem bekannt und ist Gegenstand ständiger Verbesserungen. Dennoch ergeben sich bei der Behandlung von Saatgut eine Reihe von Problemen, die nicht immer zufriedenstellend gelöst werden können. So ist es erstrebenswert, Verfahren zum Schutz des Saatguts und der keimenden Pflanze zu entwickeln, die das zusätzliche Ausbringen von Pflanzenschutzmitteln nach der Saat oder nach dem Auflaufen der Pflanzen überflüssig machen. Es ist weiterhin erstrebenswert, die Menge des eingesetzten Wirkstoffs dahingehend zu optimieren, dass das Saatgut und die keimende Pflanze vor dem Befall durch Schädlinge bestmöglich geschützt wird, ohne jedoch die Pflanze selbst durch den eingesetzten Wirkstoff zu schädigen. Insbesondere sollten Verfahren zur Behandlung von Saatgut auch die intrinsischen insektiziden Eigenschaften transgener Pflanzen einbeziehen, um einen optimalen Schutz des Saatguts und der keimenden Pflanze bei einem minimalen Aufwand an Pflanzenschutzmitteln zu erreichen.

Die vorliegende Erfindung bezieht sich daher insbesondere auch auf ein Verfahren zum Schutz von Saatgut und keimenden Pflanzen vor dem Befall von Schädlingen, indem das Saatgut mit den Verbindungen der Formel (I) behandelt wird. Die Erfindung bezieht sich ebenfalls auf die Verwendung der Verbindungen der Formel (I) zur Behandlung von Saatgut zum Schutz des Saatguts und der keimenden Pflanze vor Schädlingen. Weiterhin bezieht sich die Erfindung auf Saatgut, welches zum Schutz vor Schädlingen mit einer Verbindung der Formel (I) behandelt wurde.

Einer der Vorteile der vorliegenden Erfindung ist es, dass aufgrund der besonderen systemischen Eigenschaften der Verbindungen der Formel (I) die Behandlung des Saatguts mit diesen Verbindungen nicht nur das Saatgut selbst, sondern auch die daraus hervorgehenden Pflanzen nach dem Auflaufen vor Schädlingen schützt. Auf diese Weise kann die unmittelbare Behandlung der Kultur zum Zeitpunkt der Aussaat oder kurz danach entfallen.

Ebenso ist es als vorteilhaft anzusehen, dass die Verbindungen der Formel (I) insbesondere auch bei transgenem Saatgut eingesetzt werden können, wobei die aus diesem Saatgut hervorgehenden Pflanzen zur Expression eines gegen Schädlinge gerichteten Proteins befähigt sind. Durch die Behandlung solchen Saatguts mit den Verbindungen der Formel (I) können bestimmte Schädlinge bereits durch die Expression des z.B. insektiziden Proteins kontrolliert werden, und es zusätzlich überraschenderweise zu einer synergistischen Wirkungsergänzung mit den Verbindungen der Formel (I) kommt, was die Effektivität des Schutzes vor Schädlingsbefall noch einmal verbessert.

Die Verbindungen der Formel (I) eignen sich zum Schutz von Saatgut jeglicher Pflanzensorte wie bereits vorstehend genannt, die in der Landwirtschaft, im Gewächshaus, in Forsten, im Gartenbau oder im Weinanbau eingesetzt wird. Insbesondere handelt es sich dabei um Saatgut von Mais, Erdnuss, Canola, Raps, Mohn, Olive, Kokosnuss, Kakao, Soja, Baumwolle, Rübe (z.B. Zuckerrübe und Futterrübe), Reis, Hirse, Weizen, Gerste, Hafer, Roggen, Sonnenblume, Zuckerrohr oder Tabak. Die Verbindungen der Formel (I) eigenen sich ebenfalls zur Behandlung des Saatguts von Obstpflanzen und Gemüse wie vorstehend bereits genannt. Besondere Bedeutung kommt der Behandlung des Saatguts von Mais, Soja, Baumwolle, Weizen und Canola oder Raps zu.

Wie vorstehend bereits erwähnt, kommt auch der Behandlung von transgenem Saatgut mit Verbindungen der Formel (I) eine besondere Bedeutung zu. Dabei handelt es sich um das Saatgut von Pflanzen, die in der Regel zumindest ein heterologes Gen enthalten, das die Expression eines Polypeptids mit insbesondere insektiziden Eigenschaften steuert. Die heterologen Gene in transgenem Saatgut können dabei aus Mikroorganismen wie *Bacillus, Rhizobium, Pseudomonas, Serratia, Trichoderma, Clavibacter, Glomus* oder *Gliocladium* stammen. Die vorliegende Erfindung eignet sich besonders für die Behandlung von transgenem Saatgut, das zumindest ein heterologes Gen enthält, das aus *Bacillus sp.* stammt und dessen Genprodukt Wirksamkeit gegen Maiszünsler und/oder Maiswurzel-Bohrer zeigt. Besonders bevorzugt handelt es sich dabei um ein heterologes Gen, das aus *Bacillus thuringiensis* stammt.

Im Rahmen der vorliegenden Erfindung wird die Verbindung der Formel (I) alleine oder in einer geeigneten Formulierung auf das Saatgut aufgebracht. Vorzugsweise wird das Saatgut in einem Zustand behandelt, in dem es so stabil ist, dass keine Schäden bei der Behandlung auftreten. Im Allgemeinen kann die Behandlung des Saatguts zu jedem Zeitpunkt zwischen der Ernte und der Aussaat erfolgen. Üblicherweise wird Saatgut verwendet, das von der Pflanze getrennt und von Kolben, Schalen, Stängeln, Hülle, Wolle oder Fruchtfleisch befreit wurde.

Im Allgemeinen muss bei der Behandlung des Saatguts darauf geachtet werden, dass die Menge des auf das Saatgut aufgebrachten Verbindung der Formel (I) und/oder weiterer Zusatzstoffe so gewählt wird, dass die Keimung des Saatguts nicht beeinträchtigt bzw. die daraus hervorgehende Pflanze nicht geschädigt wird. Dies ist vor allem bei

Wirkstoffen zu beachten, die in bestimmten Aufwandmengen phytotoxische Effekte zeigen können.

Die Verbindungen der Formel (I) können unmittelbar aufgebracht werden, also ohne weitere Komponenten zu enthalten und ohne verdünnt worden zu sein. In der Regel ist es vorzuziehen, die Mittel in Form einer geeigneten Formulierung auf das Saatgut aufzubringen. Geeignete Formulierungen und Verfahren für die Saatgutbehandlung sind dem Fachmann bekannt und werden z.B. in den folgenden Dokumenten beschrieben: US 4,272,417 A, US 4,245,432 A, US 4,808,430 A, US 5,876,739 A, US 2003/0176428 A1, WO 2002/080675 A1, WO 2002/028186A2.

Die erfindungsgemäßen Saatgutbeizen eignen sich zur Bekämpfung von tierischen Schädlingen, vorzugsweise Arthropoden und Nematoden, insbesondere Insekten und Spinnentieren, die in der Landwirtschaft und in Forsten vorkommen. Sie sind gegen normal sensible und resistente Arten sowie gegen alle oder einzelne Entwicklungsstadien wirksam. Zu den oben erwähnten Schädlingen gehören:

Aus der Ordnung der Anoplura (Phthiraptera) z.B. Damalinia spp., Haematopinus spp., Linognathus spp., Pediculus spp., Trichodectes spp.

Aus der Klasse der Arachnida z.B. Acarus siro, Aceria sheldoni, Aculops spp., Aculus spp., Amblyomma spp., Argas spp., Boophilus spp., Brevipalpus spp., Bryobia praetiosa, Chorioptes spp., Dermanyssus gallinae, Eotetranychus spp., Epitrimerus pyri, Eutetranychus spp., Eriophyes spp., Hemitarsonemus spp., Hyalomma spp., Ixodes spp., Latrodectus mactans, Metatetranychus spp., Oligonychus spp., Ornithodoros spp., Panonychus spp., Phyllocoptruta oleivora, Polyphagotarsonemus latus, Psoroptes spp., Rhipicephalus spp., Rhizoglyphus spp., Sarcoptes spp., Scorpio maurus, Stenotarsonemus spp., Tarsonemus spp., Tetranychus spp., Vasates lycopersici.

Aus der Klasse der Bivalva z.B. Dreissena spp.

Aus der Ordnung der Chilopoda z.B. Geophilus spp., Scutigera spp.

Aus der Ordnung der Coleoptera z.B. Acanthoscelides obtectus, Adoretus spp., Agelastica alni, Agriotes spp., Amphimallon solstitialis, Anobium punctatum, Anoplophora spp., Anthonomus spp., Anthrenus spp., Apogonia spp., Atomaria spp., Attagenus spp., Bruchidius obtectus, Bruchus spp., Ceuthorhynchus spp., Cleonus mendicus, Conoderus spp., Cosmopolites spp., Costelytra zealandica, Curculio spp., Cryptorhynchus lapathi, Dermestes spp., Diabrotica spp., Epilachna spp., Faustinus cubae, Gibbium psylloides, Heteronychus arator, Hylamorpha elegans, Hylotrupes bajulus, Hypera postica, Hypothenemus spp., Lachnosterna consanguinea, Leptinotarsa decemlineata, Lissorhoptrus oryzophilus, Lixus spp., Lyctus spp., Meligethes aeneus, Melolontha melolontha, Migdolus spp., Monochamus spp., Naupactus xanthographus, Niptus hololeucus, Oryctes rhinoceros, Oryzaephilus surinamensis, Otiorrhynchus sulcatus, Oxycetonia jucunda, Phaedon cochleariae, Phyllophaga spp., Popillia japonica, Premnotrypes spp., Psylliodes chrysocephala, Ptinus spp., Rhizobius ventralis, Rhizopertha dominica, Sitophilus spp., Sphenophorus spp., Sternechus spp., Symphyletes spp., Tenebrio molitor, Tribolium spp., Trogoderma spp., Tychius spp., Xylotrechus spp., Zabrus spp.

Aus der Ordnung der Collembola z.B. Onychiurus armatus.

Aus der Ordnung der Dermaptera z.B. Forficula auricularia.

Aus der Ordnung der Diplopoda z.B. Blaniulus guttulatus.

Aus der Ordnung der Diptera z.B. Aedes spp., Anopheles spp., Bibio hortulanus, Calliphora erythrocephala, Ceratitis capitata, Chrysomyia spp., Cochliomyia spp., Cordylobia anthropophaga, Culex spp., Cuterebra spp., Dacus oleae, Dermatobia hominis, Drosophila spp., Fannia spp., Gastrophilus spp., Hylemyia spp., Hyppobosca spp., Hypoderma spp., Liriomyza spp.. Lucilia spp., Musca spp., Nezara spp., Oestrus spp., Oscinella frit, Pegomyia hyoscyami, Phorbia spp., Stomoxys spp., Tabanus spp., Tannia spp., Tipula paludosa, Wohlfahrtia spp.

Aus der Klasse der Gastropoda z.B. Arion spp., Biomphalaria spp., Bulinus spp., Deroceras spp., Galba spp., Lymnaea spp., Oncomelania spp., Succinea spp.

Aus der Klasse der Helminthen z.B. Ancylostoma duodenale, Ancylostoma ceylanicum, Acylostoma braziliensis, Ancylostoma spp., Ascaris lubricoides, Ascaris spp., Brugia malayi, Brugia timori, Bunostomum spp., Chabertia spp., Clonorchis spp., Cooperia spp., Dicrocoelium spp, Dictyocaulus filaria, Diphyllobothrium latum, Dracunculus medinensis, Echinococcus granulosus, Echinococcus multilocularis, Enterobius vermicularis, Faciola spp., Haemonchus spp., Heterakis spp., Hymenolepis nana, Hyostrongulus spp., Loa Loa, Nematodirus spp., Oesophagostomum

spp., Opisthorchis spp., Onchocerca volvulus, Ostertagia spp., Paragonimus spp., Schistosomen spp, Strongyloides fuelleborni, Strongyloides stercoralis, Stronyloides spp., Taenia saginata, Taenia solium, Trichinella spiralis, Trichinella nativa, Trichinella britovi, Trichinella nelsoni, Trichinella pseudopsiralis, Trichostrongulus spp., Trichuris trichuria, Wuchereria bancrofti.

Weiterhin lassen sich Protozoen, wie Eimeria, bekämpfen.

Aus der Ordnung der Heteroptera z.B. Anasa tristis, Antestiopsis spp., Blissus spp., Calocoris spp., Campylomma livida, Cavelerius spp., Cimex spp., Creontiades dilutus, Dasynus piperis, Dichelops furcatus, Diconocoris hewetti, Dysdercus spp., Euschistus spp., Eurygaster spp., Heliopeltis spp., Horcias nobilellus, Leptocorisa spp., Leptoglossus phyllopus, Lygus spp., Macropes excavatus, Miridae, Nezara spp., Oebalus spp., Pentomidae, Piesma quadrata, Piezodorus spp., Psallus seriatus, Pseudacysta persea, Rhodnius spp., Sahlbergella singularis, Scotinophora spp., Stephanitis nashi, Tibraca spp., Triatoma spp.

Aus der Ordnung der Homoptera z.B. Acyrthosipon spp., Aeneolamia spp., Agonoscena spp., Aleurodes spp., Aleurolobus barodensis, Aleurothrixus spp., Amrasca spp., Anuraphis cardui, Aonidiella spp., Aphanostigma piri, Aphis spp., Arboridia apicalis, Aspidiella spp., Aspidiotus spp., Atanus spp., Aulacorthum solani, Bemisia spp., Brachycaudus helichrysii, Brachycolus spp., Brevicoryne brassicae, Calligypona marginata, Carneocephala fulgida, Ceratovacuna lanigera, Cercopidae, Ceroplastes spp., Chaetosiphon fragaefolii, Chionaspis tegalensis, Chlorita onukii, Chromaphis juglandicola, Chrysomphalus ficus, Cicadulina mbila, Coccomytilus halli, Coccus spp., Cryptomyzus ribis, Dalbulus spp., Dialeurodes spp., Diaphorina spp., Diaspis spp., Doralis spp., Drosicha spp., Dysaphis spp., Dysmicoccus spp., Empoasca spp., Eriosoma spp., Erythroneura spp., Euscelis bilobatus, Geococcus coffeae, Homalodisca coagulata, Hyalopterus arundinis, Icerya spp., Idiocerus spp., Idioscopus spp., Laodelphax striatellus, Lecanium spp., Lepidosaphes spp., Lipaphis erysimi, Macrosiphum spp., Mahanarva fimbriolata, Melanaphis sacchari, Metcalfiella spp., Metopolophium dirhodum, Monellia costalis, Monelliopsis pecanis, Myzus spp., Nasonovia ribisnigri, Nephotettix spp., Nilaparvata lugens, Oncometopia spp., Orthezia praelonga, Parabemisia myricae, Paratrioza spp., Parlatoria spp., Pemphigus spp., Peregrinus maidis, Phenacoccus spp., Phloeomyzus passerinii, Phorodon humuli, Phylloxera spp., Pinnaspis aspidistrae, Planococcus spp., Protopulvinaria pyriformis, Pseudaulacaspis pentagona, Pseudococcus spp., Psylla spp., Pteromalus spp., Pyrilla spp., Quadraspidiotus spp., Quesada gigas, Rastrococcus spp., Rhopalosiphum spp., Saissetia spp., Scaphoides titanus, Schizaphis graminum, Selenaspidus articulatus, Sogata spp., Sogatella furcifera, Sogatodes spp., Stictocephala festina, Tenalaphara malayensis, Tinocallis caryaefoliae, Tomaspis spp., Toxoptera spp., Trialeurodes vaporariorum, Trioza spp., Typhlocyba spp., Unaspis spp., Viteus vitifolii.

Aus der Ordnung der Hymenoptera z.B. Diprion spp., Hoplocampa spp., Lasius spp., Monomorium pharaonis, Vespa spp.

Aus der Ordnung der Isopoda z.B. Armadillidium vulgare, Oniscus asellus, Porcellio scaber.

Aus der Ordnung der Isoptera z.B. Reticulitermes spp., Odontotermes spp.

Aus der Ordnung der Lepidoptera z.B. Acronicta major, Aedia leucomelas, Agrotis spp., Alabama argillacea, Anticarsia spp., Barathra brassicae, Bucculatrix thurberiella, Bupalus piniarius, Cacoecia podana, Capua reticulana, Carpocapsa pomonella, Cheimatobia brumata, Chilo spp., Choristoneura fumiferana, Clysia ambiguella, Cnaphalocerus spp., Cydia pomonella, Earias insulana, Ephestia kuehniella, Euproctis chrysorrhoea, Euxoa spp., Feltia spp., Galleria mellonella, Helicoverpa spp., Heliothis spp., Hofmannophila pseudospretella, Homona magnanima, Hyponomeuta padella, Laphygma spp., Lithocolletis blancardella, Lithophane antennata, Loxagrotis albicosta, Lymantria spp., Malacosoma neustria, Mamestra brassicae, Mocis repanda, Mythimna separata, Oria spp., Oulema oryzae, Panolis flammea, Pectinophora gossypiella, Phyllocnistis citrella, Pieris spp., Plutella xylostella, Prodenia spp., Pseudaletia spp., Pseudoplusia includens, Pyrausta nubilalis, Spodoptera spp., Thermesia gemmatalis, Tinea pellionella, Tineola bisselliella, Tortrix viridana, Trichoplusia spp.

Aus der Ordnung der Orthoptera z.B. Acheta domesticus, Blatta orientalis, Blattella germanica, Gryllotalpa spp., Leucophaea maderae, Locusta spp., Melanoplus spp., Periplaneta americana, Schistocerca gregaria.

Aus der Ordnung der Siphonaptera z.B. Ceratophyllus spp., Xenopsylla cheopis.

Aus der Ordnung der Symphyla z.B. Scutigerella immaculata.

Aus der Ordnung der Thysanoptera z.B. Baliothrips biformis, Enneothrips flavens, Frankliniella spp., Heliothrips spp., Hercinothrips femoralis, Kakothrips spp., Rhipiphorothrips cruentatus, Scirtothrips spp., Taeniothrips cardamoni, Thrips spp.

Aus der Ordnung der Thysanura z.B. Lepisma saccharina.

Zu den pflanzenparasitären Nematoden gehören z.B. Anguina spp., Aphelenchoides spp., Belonoaimus spp., Bursaphelenchus spp., Ditylenchus dipsaci, Globodera spp., Heliocotylenchus spp., Heterodera spp., Longidorus spp., Meloidogyne spp., Pratylenchus spp., Radopholus similis, Rotylenchus spp., Trichodorus spp., Tylenchorhynchus spp., Tylenchulus spp., Tylenchulus semipenetrans, Xiphinema spp.

Mit Hilfe der erfindungsgemäßen Beize sind vorzugsweise Insekten folgender Ordnungen bekämpfbar:

Bodeninsekten: Diptera (z.B. Fritfliege, Brachfliege), Coleoptera (z.B. Diabrotica, Drahtwurm), Lepidoptera (z.B. Saateule), Blattophtheroidea, Myriopoda.

Blattinsekten: Aphidina, Coleoptera, Brachycera, Lepidotera, Homoptera, Tysanoptera, Aleurodina, Cicadina, Acasi, Cossina, Heteroptera.

Überraschenderweise wurde ferner gefunden, dass die Verbindungen der Formel (I) systemische Eigenschaften besitzen und über den Boden ausgebracht eine sehr gute Wirkung gegen die oben genannten tierischen Schädlinge aufweisen.

Hier wird vorteilhaft Granulat, welches den oder die Wirkstoffe enthält, in oder auf den Boden ausgebracht. In Frage kommen beispielsweise broadcast-, Band-, Furchen- und Pflanzlochapplikation. Unter broadcast-applikation versteht man das oberflächige Ausbringen des Wirkstoffs über die gesamte zu behandelnde Fläche mit einer anschließenden mechanischen Einarbeitung in den Boden.

Insbesondere sei die Anwendung in Pflanzkästen (Saatkästen) im Reisanbau erwähnt (nursery box treatment).

Besonders vorteilhaft ist es, die Verbindungen der Formel (I) oder ihre Salze in Wasser zu emulgieren oder zu lösen und dieses zur Bewässerung der Pflanzen zu verwenden.

In Frage kommen beispielsweise Spritzungen auf den Boden, Drenching, d.h. das Angießen der Pflanzen mit wirkstoffhaltigen Lösungen und Tröpfchenbewässerung (Drip-Irrigation), sowie die Anwendung in Hydrokulturen, insbesondere im Gemüse- und Zierpflanzenanbau.

Die Verbindungen der Formel (I) können auch über den Stamm appliziert werden, beispielsweise durch eine Stamminjektion.

Ferner wurde gefunden, dass sich die Verbindungen der Formel (I) insbesondere hervorragend zur Bekämpfung der Stubenfliege eignen.

Die Verbindung der Formel (I) kann erfindungsgemäß bei der Bekämpfung von Schaben, also Insekten der Ordnung Blattariae, insbesondere der Familie Blattellidae, vorzugsweise der Art Blattella germanica oder der Familie Blattidae, vorzugsweise der Arten Blatta orientalis und Periplaneta americana, aber auch gegen andere Schabenarten, ganz besonders bevorzugt jedoch gegen Blattella germanica, eingesetzt werden.

Die Verbindung der Formel (I) wirkt erfindungsgemäß auf die Schaben derart, dass die Repellentwirkung von Insektiziden, z.B. von Pyrethroiden, verringert wird.

Dieser Effekt tritt bei allen beweglichen Entwicklungsstadien (Larven, Adulte) der Schaben auf. Die para-Hydroxyphenylessigsäure und/oder ihre Mischungen mit anderen chemischen Verbindungen können somit ganz allgemein bei der Schabenbekämpfung, unabhängig von der Art der angewandten Bekämpfungsmethode eingesetzt werden. Sie kann bevorzugt bei chemischen Bekämpfungsverfahren und gegebenenfalls zusammen mit weiteren wirksamen Mitteln, wie anlockenden Ködermaterialien oder anderen Lockmitteln, synthetischen oder natürlichen Insektiziden usw. angewandt werden.

Dem Fachmann ist es anhand einfacher Überlegungen oder einfacher Untersuchungen leicht möglich, die für die jeweiligen Verwendungszwecke günstigen Mischungen sowie Anwendungsarten und Mengen zu ermitteln.

Es wurde weiterhin gefunden, dass sich die bekannten Verbindungen der Formel (I) hervorragend auch zur Bekämpfung von Schädlingen eignen, die nicht in der EP 0 539 588 genannt (Heerwurm, Kohlmotte, Blattläuse, Zikaden und die braune Reiszikade) sind.

Bevorzugt lassen sich die Verbindungen der Formel (I) zur Bekämpfung der in den Beispielen genannten Schädlinge verwenden.

Es wurde weiter gefunden, dass die Verbindungen der Formel (I) nicht nur gegen Pflanzen-, Hygiene- und Vorratsschädlinge wirken, sondern auch auf dem veterinärmedizinischen Sektor gegen tierische Parasiten (Ektoparasiten) wie Schildzecken, Lederzecken, Räudemilben, Laufmilben, Fliegen (stechend und leckend), parasitierende Fliegenlarven, Läuse, Haarlinge, Federlinge und Flöhe. Zu diesen Parasiten gehören:

Aus der Ordnung der Anoplurida z.B. Haematopinus spp., Linognathus spp., Pediculus spp., Phtirus spp., Solenopotes spp.

Aus der Ordnung der Mallophagida und den Unterordnungen Amblycerina sowie Ischnocerina z.B. Trimenopon spp., Menopon spp., Trinoton spp., Bovicola spp., Werneckiella spp., Lepikentron spp., Damalina spp., Trichodectes spp., Felicola spp.

Aus der Ordnung Diptera und den Unterordnungen Nematocerina sowie Brachycerina z.B. Aedes spp., Anopheles spp., Culex spp., Simulium spp., Eusimulium spp., Phlebotomus spp., Lutzomyia spp., Culicoides spp., Chrysops spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp.

Aus der Ordnung der Siphonapterida z.B. Pulex spp., Ctenocephalides spp., Xenopsylla spp., Ceratophyllus spp.

Aus der Ordnung der Heteropterida z.B. Cimex spp., Triatoma spp., Rhodnius spp., Panstrongylus spp.

Aus der Ordnung der Blattarida z.B. Blatta orientalis, Periplaneta americana, Blattela germanica, Supella spp.

Aus der Unterklasse der Acari (Acarina) und den Ordnungen der Meta- sowie Mesostigmata z.B. Argas spp., Ornithodorus spp., Otobius spp., Ixodes spp., Amblyomma spp., Boophilus spp., Dermacentor spp., Haemophysalis spp., Hyalomma spp., Rhipicephalus spp., Dermanyssus spp., Raillietia spp., Pneumonyssus spp., Sternostoma spp., Varroa spp.

Aus der Ordnung der Actinedida (Prostigmata) und Acaridida (Astigmata) z.B. Acarapis spp., Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp.

Die Verbindungen der Formel (I) eignen sich auch zur Bekämpfung von Arthropoden, die landwirtschaftliche Nutztiere, wie z.B. Rinder, Schafe, Ziegen, Pferde, Schweine, Esel, Kamele, Büffel, Kaninchen, Hühner, Puten, Enten, Gänse, Bienen, sonstige Haustiere wie z.B. Hunde, Katzen, Stubenvögel, Aquarienfische sowie sogenannte Versuchstiere, wie z.B. Hamster, Meerschweinchen, Ratten und Mäuse befallen. Durch die Bekämpfung dieser Arthropoden sollen Todesfälle und Leistungsminderungen (bei Fleisch, Milch, Wolle, Häuten, Eiern, Honig usw.) vermindert werden, so dass durch den Einsatz der erfindungsgemäßen Wirkstoffkombinationen eine wirtschaftlichere und einfachere Tierhaltung möglich ist.

Die Anwendung der Verbindungen der Formel (I) geschieht im Veterinärsektor in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subcutan, intravenös, intraperitonel u.a.), Implantate, durch nasale Applikation, durch dermale Anwendung in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen Formkörpern, wie Halsbändern, Ohrmarken, Schwanzmarken, Gliedmaßenbändern, Halftern, Markierungsvorrichtungen usw.

Bei der Anwendung für Vieh, Geflügel, Haustiere etc. kann man die Wirkstoffe als Formulierungen (beispielsweise Pulver, Emulsionen, fließfähige Mittel), die die Wirkstoffe in einer Menge von 1 bis 80 Gew.-% enthalten, direkt oder nach 100 bis 10 000-facher Verdünnung anwenden oder sie als chemisches Bad verwenden.

Außerdem wurde gefunden, dass die Verbindungen der Formel (I) eine hohe insektizide Wirkung gegen Insekten zeigen, die technische Materialien zerstören.

Beispielhaft und vorzugsweise - ohne jedoch zu limitieren - seien die folgenden Insekten genannt: Käfer wie

Hylotrupes bajulus, Chlorophorus pilosis, Anobium punctatum, Xestobium rufovillosum, Ptilinus pecticornis, Dendrobium pertinex, Ernobius mollis, Priobium carpini, Lyctus brunneus, Lyctus africanus, Lyctus planicollis, Lyctus linearis, Lyctus pubescens, Trogoxylon aequale, Minthes rugicollis, Xyleborus spec. Tryptodendron spec. Apate monachus, Bostrychus capucins, Heterobostrychus brunneus, Sinoxylon spec. Dinoderus minutus.

Hautflügler wie

Sirex juvencus, Urocerus gigas, Urocerus gigas taignus, Urocerus augur.

Termiten wie

Kalotermes flavicollis, Cryptotermes brevis, Heterotermes indicola, Reticulitermes flavipes, Reticulitermes santonensis, Reticulitermes lucifugus, Mastotermes darwiniensis, Zootermopsis nevadensis, Coptotermes formosanus, Odontotermes formosanus, Odontotermes lokanandi, Odontotermes obesus, Odontotermes smeathmani.

Borstenschwänze wie Lepisma saccharina.

Unter technischen Materialien sind im vorliegenden Zusammenhang nicht-lebende Materialien zu verstehen, wie vorzugsweise Kunststoffe, Klebstoffe, Leime, Papiere und Kartone, Leder, Holz, Holzverarbeitungsprodukte und Anstrichmittel.

Ganz besonders bevorzugt handelt es sich bei dem vor Insektenbefall zu schützenden Material um Holz und Holzverarbeitungsprodukte.

Unter Holz und Holzverarbeitungsprodukten, welche durch das erfindungsgemäße Mittel bzw. dieses enthaltende Mischungen geschützt werden kann, ist beispielhaft zu verstehen:

Bauholz, Holzbalken, Eisenbahnschwellen, Brückenteile, Bootsstege, Holzfahrzeuge, Kisten, Paletten, Container, Telefonmasten, Holzverkleidungen, Holzfenster und -türen, Sperrholz, Spanplatten, Tischlerarbeiten oder Holzprodukte, die ganz allgemein beim Hausbau oder in der Bautischlerei Verwendung finden.

Die Verbindungen der Formel (I) können als solche, in Form von Konzentraten oder allgemein üblichen Formulierungen wie Pulver, Granulate, Lösungen, Suspensionen, Emulsionen oder Pasten angewendet werden.

Die genannten Formulierungen können in an sich bekannter Weise hergestellt werden, z.B. durch Vermischen der Wirkstoffe mit mindestens einem Lösungs- bzw. Verdünnungsmittel, Emulgator, Dispergier- und/oder Binde- oder Fixiermittels, Wasser-Repellent, gegebenenfalls Sikkative und UV-Stabilisatoren und gegebenenfalls Farbstoffen und Pigmenten sowie weiteren Verarbeitungshilfsmitteln.

Die zum Schutz von Holz und Holzwerkstoffen verwendeten insektiziden Mittel oder Konzentrate enthalten den erfindungsgemäßen Wirkstoff in einer Konzentration von 0,0001 bis 95 Gew.-%, insbesondere 0,001 bis 60 Gew.-%.

Die Menge der eingesetzten Mittel bzw. Konzentrate ist von der Art und dem Vorkommen der Insekten und von dem Medium abhängig. Die optimale Einsatzmenge kann bei der Anwendung jeweils durch Testreihen ermittelt werden. Im allgemeinen ist es jedoch ausreichend 0,0001 bis 20 Gew.-%, vorzugsweise 0,001 bis 10 Gew.-%, des Wirkstoffs, bezogen auf das zu schützende Material, einzusetzen.

Als Lösungs- und/oder Verdünnungsmittel dient ein organisch-chemisches Lösungsmittel oder Lösungsmittelgemisch und/oder ein öliges oder ölartiges schwer flüchtiges organisch-chemisches Lösungsmittel oder Lösungsmittelgemisch und/oder ein polares organisch-chemisches Lösungsmittel oder Lösungsmittelgemisch und/oder Wasser und gegebenenfalls einen Emulgator und/oder Netzmittel.

Als organisch-chemische Lösungsmittel werden vorzugsweise ölige oder ölartige Lösungsmittel mit einer Verdunstungszahl über 35 und einem Flammpunkt oberhalb 30˚C, vorzugsweise oberhalb 45˚C, eingesetzt. Als derartige schwerflüchtige, wasserunlösliche, ölige und ölartige Lösungsmittel werden entsprechende Mineralöle oder deren Aromatenfraktionen oder mineralölhaltige Lösungsmittelgemische, vorzugsweise Testbenzin, Petroleum und/oder Alkylbenzol verwendet.

Vorteilhaft gelangen Mineralöle mit einem Siedebereich von 170 bis 220˚C, Testbenzin mit einem Siedebereich von 170 bis 220˚C, Spindelöl mit einem Siedebereich von 250 bis 350˚C, Petroleum bzw. Aromaten vom Siedebereich von 160 bis 280˚C, Terpentinöl und dgl. zum Einsatz.

In einer bevorzugten Ausführungsform werden flüssige aliphatische Kohlenwasserstoffe mit einem Siedebereich von 180 bis 210˚C oder hochsiedende Gemische von aromatischen und aliphatischen Kohlenwasserstoffen mit einem Siedebereich von 180 bis 220˚C und/oder Spindeöl und/oder Monochlornaphthalin, vorzugsweise $\alpha$-Monochlornaphthalin, verwendet.

Die organischen schwerflüchtigen öligen oder ölartigen Lösungsmittel mit einer Verdunstungszahl über 35 und einem Flammpunkt oberhalb 30˚C, vorzugsweise oberhalb 45˚C, können teilweise durch leicht oder mittelflüchtige organisch-chemische Lösungsmittel ersetzt werden, mit der Maßgabe, dass das Lösungsmittelgemisch ebenfalls eine Verdunstungszahl über 35 und einen Flammpunkt oberhalb 30˚C, vorzugsweise oberhalb 45˚C, aufweist und dass das Insektizid-FungizidGemisch in diesem Lösungsmittelgemisch löslich oder emulgierbar ist.

Nach einer bevorzugten Ausführungsform wird ein Teil des organisch-chemischen Lösungsmittel oder Lösungsmittelgemisches durch ein aliphatisches polares organisch-chemisches Lösungsmittel oder Lösungsmittelgemisch ersetzt. Vorzugsweise gelangen Hydroxyl- und/oder Ester- und/oder Ethergruppen enthaltende aliphatische organisch-chemische Lösungsmittel wie beispielsweise Glycolether, Ester oder dgl. zur Anwendung.

Als organisch-chemische Bindemittel werden im Rahmen der vorliegenden Erfindung die an sich bekannten wasserverdünnbaren und/oder in den eingesetzten organisch-chemischen Lösungsmitteln löslichen oder dispergier- bzw. emulgierbaren Kunstharze und/oder bindende trocknende Öle, insbesondere Bindemittel bestehend aus oder enthaltend ein Acrylatharz, ein Vinylharz, z.B. Polyvinylacetat, Polyesterharz, Polykondensations- oder Polyadditionsharz, Polyurethanharz, Alkydharz bzw. modifiziertes Alkydharz, Phenolharz, Kohlenwasserstoffharz wie Inden-Cumaronharz, Siliconharz, trocknende pflanzliche und/oder trocknende Öle und/oder physikalisch trocknende Bindemittel auf der Basis eines Natur- und/oder Kunstharzes verwendet.

Das als Bindemittel verwendete Kunstharz kann in Form einer Emulsion, Dispersion oder Lösung, eingesetzt werden. Als Bindemittel können auch Bitumen oder bituminöse Substanzen bis zu 10 Gew.-%, verwendet werden. Zusätzlich können an sich bekannte Farbstoffe, Pigmente, wasserabweisende Mittel, Geruchskorrigentien und Inhibitoren bzw. Korrosionsschutzmittel und dgl. eingesetzt werden.

Bevorzugt ist gemäß der Erfindung als organisch-chemische Bindemittel mindestens ein Alkydharz bzw. modifiziertes Alkydharz und/oder ein trocknendes pflanzliches Öl im Mittel oder im Konzentrat enthalten. Bevorzugt werden gemäß der Erfindung Alkydharze mit einem Ölgehalt von mehr als 45 Gew.-%, vorzugsweise 50 bis 68 Gew.-%, verwendet.

Das erwähnte Bindemittel kann ganz oder teilweise durch ein Fixierungsmittel(gemisch) oder ein Weichmacher (gemisch) ersetzt werden. Diese Zusätze sollen einer Verflüchtigung der Wirkstoffe sowie einer Kristallisation bzw. Ausfällem vorbeugen. Vorzugsweise ersetzen sie 0,01 bis 30 % des Bindemittels (bezogen auf 100 % des eingesetzten Bindemittels).

Die Weichmacher stammen aus den chemischen Klassen der Phthalsäureester wie Dibutyl-, Dioctyl- oder Benzylbutylphthalat, Phosphorsäureester wie Tributylphosphat, Adipinsäureester wie Di-(2-ethylhexyl)-adipat, Stearate wie Butylstearat oder Amylstearat, Oleate wie Butyloleat, Glycerinether oder höhermolekulare Glykolether, Glycerinester sowie p-Toluolsulfonsäureester.

Fixierungsmittel basieren chemisch auf Polyvinylalkylethern wie z.B. Polyvinylmethylether oder Ketonen wie Benzophenon, Ethylenbenzophenon.

Als Lösungs- bzw. Verdünnungsmittel kommt insbesondere auch Wasser in Frage, gegebenenfalls in Mischung mit einem oder mehreren der oben genannten organisch-chemischen Lösungs- bzw. Verdünnungsmittel, Emulgatoren und Dispergatoren.

Ein besonders effektiver Holzschutz wird durch großtechnische Imprägnierverfahren, z.B. Vakuum, Doppelvakuum oder Druckverfahren, erzielt.

Die anwendungsfertigen Mittel können gegebenenfalls noch weitere Insektizide und gegebenenfalls noch ein oder mehrere Fungizide enthalten.

Zugleich können die Verbindungen der Formel (I) zum Schutz vor Bewuchs von Gegenständen, insbesondere von Schiffskörpern, Sieben, Netzen, Bauwerken, Kaianlagen und Signalanlagen, welche mit See- oder Brackwasser in Verbindung kommen, eingesetzt werden.

Bewuchs durch sessile Oligochaeten, wie Kalkröhrenwürmer sowie durch Muscheln und Arten der Gruppe Ledamorpha (Entenmuscheln), wie verschiedene Lepas- und Scalpellum-Arten, oder durch Arten der Gruppe Balanomorpha (Seepocken), wie Balanus- oder Pollicipes-Species, erhöht den Reibungswiderstand von Schiffen und führt in der Folge durch erhöhten Energieverbrauch und darüber hinaus durch häufige Trockendockaufenthalte zu einer deutlichen Steigerung der Betriebskosten.

Neben dem Bewuchs durch Algen, beispielsweise Ectocarpus sp. und Ceramium sp., kommt insbesondere dem Bewuchs durch sessile Entomostraken-Gruppen, welche unter dem Namen Cirripedia (Rankenflusskrebse) zusammengefasst werden, besondere Bedeutung zu.

Es wurde nun überraschenderweise gefunden, dass die erfindungsgemäßen Wirkstoffkombinationen eine hervorragende Antifouling (Antibewuchs)-Wirkung aufweisen.

Durch Einsatz der Verbindungen der Formel (I) kann auf den Einsatz von Schwermetallen wie z.B. in Bis (trialkylzinn)-sulfiden, Tri-*n*-butylzinnlaurat, Tri-*n*-butylzinnchlorid, Kupfer(I)-oxid, Triethylzinnchlorid, Tri-*n*-butyl(2-phenyl-4-chlorphenoxy)-zinn, Tributylzinnoxid, Molybdändisulfid, Antimonoxid, polymerem Butyltitanat, Phenyl-(bispyridin)-wismutchlorid, Tri-*n*-butylzinnfluorid, Manganethylenbisthiocarbamat, Zinkdimethyldithiocarbamat, Zinkethylenbisthiocarbamat, Zink- und Kupfersalze von 2-Pyridinthiol-1-oxid, Bisdimethyldithiocarbamoylzinkethylenbisthiocarbamat, Zinkoxid, Kupfer(I)-ethylen-bisdithiocarbamat, Kupferthiocyanat, Kupfernaphthenat und Tributylzinnhalogeniden verzichtet werden oder die Konzentration dieser Verbindungen entscheidend reduziert werden.

Die anwendungsfertigen Antifoulingfarben können gegebenenfalls noch andere Wirkstoffe, vorzugsweise Algizide, Fungizide, Herbizide, Molluskizide bzw. andere Antifouling-Wirkstoffe enthalten.

Als Kombinationspartner für die erfindungsgemäßen Antifouling-Mittel eignen sich vorzugsweise:

Algizide wie

2-*tert*.-Butylamino-4-cyclopropylamino-6-methylthio-1,3,5-triazin, Dichlorophen, Diuron, Endothal, Fentinacetat, Isoproturon, Methabenzthiazuron, Oxyfluorfen, Quinoclamine und Terbutryn;

Fungizide wie

Benzo[*b*]thiophencarbonsäurecyclohexylamid-S,S-dioxid, Dichlofluanid, Fluorfolpet, 3-Iod-2-propinyl-butylcarbamat, Tolylfluanid und Azole wie

Azaconazole, Cyproconazole, Epoxyconazole, Hexaconazole, Metconazole, Propiconazole und Tebuconazole;

Molluskizide wie Fe-Komplex-Bildner, Fentinacetat, Metaldehyd, Methiocarb, Niclosamid, Ethiprole und Trimethacarb;

oder herkömmliche Antifouling-Wirkstoffe wie

4,5-Dichlor-2-octyl-4-isothiazolin-3-on, Diiodmethylparatrylsulfon, 2-(N,N-Dimethylthiocarbamoylthio)-5-nitrothiazyl, Kalium-, Kupfer-, Natrium- und Zinksalze von 2-Pyridinthiol-1-oxid, Pyridin-triphenylboran, Tetrabutyldistannoxan, 2,3,5,6-Tetrachlor-4-(methylsulfonyl)-pyridin, 2,4,5,6-Tetrachloroisophthalonitril, Tetramethylthiuramdisulfid und 2,4,6-Trichlorphenylmaleinimid.

Die verwendeten Antifouling-Mittel enthalten den Wirkstoff in einer Konzentration von 0,001 bis 50 Gew.-%, insbesondere von 0,01 bis 20 Gew.-%.

Die Antifouling-Mittel enthalten desweiteren die üblichen Bestandteile wie z.B. in Ungerer, *Chem. Ind.* **1985**, *37*, 730-732 und Williams, Antifouling Marine Coatings, Noyes, Park Ridge, **1973** beschrieben.

Antifouling-Anstrichmittel enthalten neben den algiziden, fungiziden, molluskiziden und insektiziden Wirkstoffen insbesondere Bindemittel.

Beispiele für anerkannte Bindemittel sind Polyvinylchlorid in einem Lösungsmittelsystem, chlorierter Kautschuk in einem Lösungsmittelsystem, Acrylharze in einem Lösungsmittelsystem insbesondere in einem wässrigen System, Vinylchlorid/Vinylacetat-Copolymersysteme in Form wässriger Dispersionen oder in Form von organischen Lösungsmittelsystemen, Butadien/Styrol/- Acrylnitril-Kautschuke, trocknende Öle, wie Leinsamenöl, Harzester oder modifizierte Hartharze in Kombination mit Teer oder Bitumina, Asphalt sowie Epoxyverbindungen, geringe Mengen Chlorkautschuk, chloriertes Polypropylen und Vinylharze.

Gegebenenfalls enthalten Anstrichmittel auch anorganische Pigmente, organische Pigmente oder Farbstoffe, welche vorzugsweise in Seewasser unlöslich sind. Ferner können Anstrichmittel Materialien, wie Kolophonium enthalten, um eine gesteuerte Freisetzung der Wirkstoffe zu ermöglichen. Die Anstriche können ferner Weichmacher, die rheologischen Eigenschaften beeinflussende Modifizierungsmittel sowie andere herkömmliche Bestandteile enthalten. Auch in Self-Polishing-Antifouling-Systemen können die erfindungsgemäßen Wirkstoffkombinationen eingearbeitet werden.

Die Verbindungen der Formel (I) eignen sich auch zur Bekämpfung von tierischen Schädlingen, insbesondere von Insekten, Spinnentieren und Milben, die in geschlossenen Räumen, wie beispielsweise Wohnungen, Fabrikhallen, Büros, Fahrzeugkabinen u.ä. vorkommen. Sie können zur Bekämpfung dieser Schädlinge allein oder in Kombination mit anderen Wirk- und Hilfsstoffen in Haushaltsinsektizid-Produkten verwendet werden. Sie sind gegen sensible und resistente Arten sowie gegen alle Entwicklungsstadien wirksam. Zu diesen Schädlingen gehören:

Aus der Ordnung der Scorpionidea z.B. Buthus occitanus.

Aus der Ordnung der Acarina z.B. Argas persicus, Argas reflexus, Bryobia ssp., Dermanyssus gallinae, Glyciphagus domesticus, Ornithodorus moubat, Rhipicephalus sanguineus, Trombicula alfreddugesi, Neutrombicula autumnalis, Dermatophagoides pteronissimus, Dermatophagoides forinae.

Aus der Ordnung der Araneae z.B. Aviculariidae, Araneidae.

Aus der Ordnung der Opiliones z.B. Pseudoscorpiones chelifer, Pseudoscorpiones cheiridium, Opiliones phalangium.

Aus der Ordnung der Isopoda z.B. Oniscus asellus, Porcellio scaber.

Aus der Ordnung der Diplopoda z.B. Blaniulus guttulatus, Polydesmus spp.

Aus der Ordnung der Chilopoda z.B. Geophilus spp.

Aus der Ordnung der Zygentoma z.B. Ctenolepisma spp., Lepisma saccharina, Lepismodes inquilinus.

Aus der Ordnung der Blattaria z.B. Blatta orientalies, Blattella germanica, Blattella asahinai, Leucophaea maderae, Panchlora spp., Parcoblatta spp., Periplaneta australasiae, Periplaneta americana, Periplaneta brunnea, Periplaneta fuliginosa, Supella longipalpa.

Aus der Ordnung der Saltatoria z.B. Acheta domesticus.

Aus der Ordnung der Dermaptera z.B. Forficula auricularia.

Aus der Ordnung der Isoptera z.B. Kalotermes spp., Reticulitermes spp.

Aus der Ordnung der Psocoptera z.B. Lepinatus spp., Liposcelis spp.

Aus der Ordnung der Coleptera z.B. Anthrenus spp., Attagenus spp., Dermestes spp., Latheticus oryzae, Necrobia spp., Ptinus spp., Rhizopertha dominica, Sitophilus granarius, Sitophilus oryzae, Sitophilus zeamais, Stegobium paniceum.

Aus der Ordnung der Diptera z.B. Aedes aegypti, Aedes albopictus, Aedes taeniorhynchus, Anopheles spp., Calliphora erythrocephala, Chrysozona pluvialis, Culex quinquefasciatus, Culex pipiens, Culex tarsalis, Drosophila spp., Fannia canicularis, Musca domestica, Phlebotomus spp., Sarcophaga carnaria, Simulium spp., Stomoxys calcitrans, Tipula paludosa.

Aus der Ordnung der Lepidoptera z.B. Achroia grisella, Galleria mellonella, Plodia interpunctella, Tinea cloacella, Tinea pellionella, Tineola bisselliella.

Aus der Ordnung der Siphonaptera z.B. Ctenocephalides canis, Ctenocephalides felis, Pulex irritans, Tunga penetrans, Xenopsylla cheopis.

Aus der Ordnung der Hymenoptera z.B. Camponotus herculeanus, Lasius fuliginosus, Lasius niger, Lasius umbratus, Monomorium pharaonis, Paravespula spp., Tetramorium caespitum.

Aus der Ordnung der Anoplura z.B. Pediculus humanus capitis, Pediculus humanus corporis, Phthirus pubis.

Aus der Ordnung der Heteroptera z.B. Cimex hemipterus, Cimex lectularius, Rhodinus prolixus, Triatoma infestans.

Die Anwendung im Bereich der Haushaltsinsektizide kann auch in Kombination mit anderen geeigneten Wirkstoffen wie Phosphorsäureestern, Carbamaten, Pyrethroiden, Wachstumsregulatoren oder Wirkstoffen aus anderen bekannten Insektizidklassen erfolgen.

Die Anwendung erfolgt in Aerosolen, drucklosen Sprühmitteln, z.B. Pump- und Zerstäubersprays, Nebelautomaten, Foggern, Schäumen, Gelen, Verdampferprodukten mit Verdampferplättchen aus Cellulose oder Kunststoff, Flüssigverdampfern, Gel- und Membranverdampfern, propellergetriebenen Verdampfern, energielosen bzw. passiven Verdampfungssystemen, Mottenpapieren, Mottensäckchen und Mottengelen als Granulate oder Stäube, in Streuködern oder Köderstationen.

Beim Einsatz der erfindungsgemäßen Wirkstoffkombinationen können die Aufwandmengen je nach Applikationsart innerhalb eines größeren Bereichs variiert werden. Bei der Behandlung von Pflanzenteilen liegen die Aufwandmengen an Wirkstoffkombination im allgemeinen zwischen 0,1 und 10 000 g/ha, vorzugsweise zwischen 10 und 1 000 g/ha.

Die gute insektizide Wirkung der Verbindungen der Formel (I) geht aus den nachfolgenden Beispielen hervor.

<u>Beispiel A</u>

**Bemisia tabaci -Test** (normal sensibler Stamm)

| | |
|---|---|
| Lösungsmittel: | 7 Gewichtsteile Dimethylformamid |
| Emulgator: | 10 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Baumwollpflanzen *(Gossypium hirsutum),* die von Eiern, Larven und Puparien der Weißen Fliege (*Bemisia tabaci*) befallen sind, werden durch Sprühen mit der Wirkstoffzubereitung in der gewünschten Konzentration behandelt.

Nach der gewünschten Zeit wird die Abtötung in % bestimmt. Dabei bedeutet 100 %, dass alle Tiere abgetötet wurden; 0 % bedeutet, dass keine Tiere abgetötet wurden.

Bei diesem Test zeigt z. B. die folgende Verbindung der Herstellungsbeispiele gute Wirksamkeit:

Tabelle A

pflanzenschädigende Insekten **Bemisia tabaci -Test** (normal sensibler Stamm)

| Wirkstoffe | Wirkstoffkonzentration in ppm | Abtötungsgrad in % nach 14$^d$ |
|---|---|---|
| 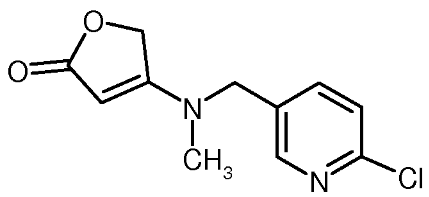 | | |
| erfindungsgemäß (84) | 100 | 100 |

Beispiel B

**Bemisia tabaci -Test** (resistenter Stamm)

| | |
|---|---|
| Lösungsmittel: | 10 Gewichtsteile Aceton |
| Emulgator: | 0,2 Gewichtsteile Triton X-100 |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Blattscheiben von Baumwollpflanzen *(Gossypium hirsutum)* werden durch Tauchen in die Wirkstoffzubereitung der gewünschten Konzentration behandelt und nach dem Antrocknen des Belages mit Adulten der Weißen Fliege (*Bemisia tabaci,* resistenter Stamm) besetzt.

Nach der gewünschten Zeit wird die Abtötung in % bestimmt. Dabei bedeutet 100 %, dass alle Tiere abgetötet wurden; 0 % bedeutet, dass keine Tiere abgetötet wurden.

Bei diesem Test zeigt z. B. die folgende Verbindung der Herstellungsbeispiele gute Wirksamkeit:

Tabelle B

pflanzenschädigende Insekten **Bemisia tabaci -Test** (resistenter Stamm)

| Wirkstoffe | Wirkstoffkonzentration in ppm | Abtötungsgrad in % nach 3$^d$ |
|---|---|---|
| 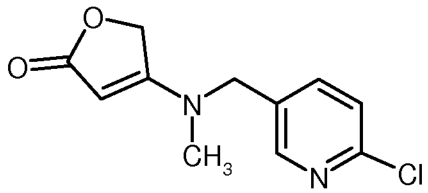 | | |

(fortgesetzt)

| pflanzenschädigende Insekten **Bemisia tabaci -Test** (resistenter Stamm) | | |
|---|---|---|
| Wirkstoffe | Wirkstoffkonzentration in ppm | Abtötungsgrad in % nach 3[d] |
| erfindungsgemäß (84) | 1000 | 78 |

Beispiel C

**Liriomyza trifolii -Test**

Lösungsmittel: 7 Gewichtsteile Dimethylformamid
Emulgator: 10 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Buschbohnenpflanzen (*Phaseolus vulgaris*), die mit Larven der Minierfliege (*Liriomyza trifolii*) befallen sind, werden durch Spritzen mit der Wirkstoffzubereitung in der gewünschten Konzentration behandelt.

Nach der gewünschten Zeit wird die Wirkung in % bestimmt. Dabei bedeutet 100 %, dass keine Miniergänge zu sehen sind; 0 % bedeutet, dass die Versuchspflanzen mit der Kontrolle vergleichbar sind.

Bei diesem Test zeigen z. B. die folgenden Verbindungen der Herstellungsbeispiele gute Wirksamkeit:

Tabelle C

pflanzenschädigende Insekten **Liriomyza trifolii - Test**

| Wirkstoffe | Wirkstoffkonzentration in ppm | Wirkung in % nach 7[d] |
|---|---|---|
| 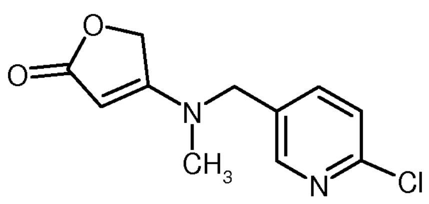 erfindungsgemäß (84) | 100 | 46 |
| 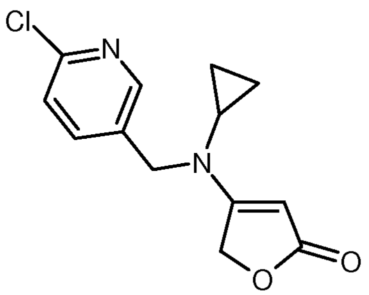 erfindungsgemäß (88) | 100 | 55 |

Beispiel D

**Frankliniella occidentalis -Test**

Lösungsmittel: 7 Gewichtsteile Dimethylformamid
Emulgator: 10 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Baumwollpflanzen *(Gossypium hirsutum)* werden durch Spritzen mit der Wirkstoffzubereitung in der gewünschten Konzentration behandelt und mit einer gemischten Thripspopulation (*Frankliniella occidentalis)* infiziert.

Nach der gewünschten Zeit wird die Wirkung in % bestimmt. Dabei bedeutet 100 %, dass alle Thripse abgetötet wurden; 0 % bedeutet, dass keine Tiere abgetötet wurden.

Bei diesem Test zeigen z. B. die folgenden Verbindungen der Herstellungsbeispiele gute Wirksamkeit:

Tabelle D

pflanzenschädigende Insekten **Frankliniella occidentalis - Test**

| Wirkstoffe | Wirkstoffkonzentration in ppm | Wirkung in % nach 14[d] |
|---|---|---|
| 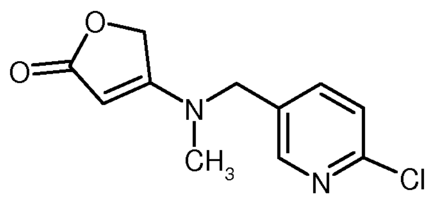 | | |
| erfindungsgemäß (84) | 100 | 99 |
| 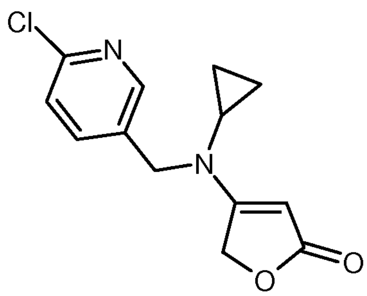 | | |
| erfindungsgemäß (88) | 100 | 99 |

Beispiel E

**Leptinotarsa decemlineata - Larven -Test**

| | |
|---|---|
| Lösungsmittel: | 7 Gewichtsteile Dimethylformamid |
| Emulgator: | 10 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Blätter von Kartoffelpflanzen (*Solanum tuberosum*), die mit Larven des Kartoffelkäfers (*Leptinotarsa decemlineata*) besetzt sind, werden durch Spritzen mit der Wirkstoffzubereitung in der gewünschten Konzentration behandelt.

Nach der gewünschten Zeit wird die Wirkung in % bestimmt. Dabei bedeutet 100 %, dass alle Käferlarven abgetötet wurden; 0 % bedeutet, dass keine Käferlarven abgetötet wurden.

Bei diesem Test zeigen z. B. die folgenden Verbindungen der Herstellungsbeispiele gute Wirksamkeit:

Tabelle E

| pflanzenschädigende Insekten **Leptinotarsa decemlineata - Larven - Test** | | |
|---|---|---|
| Wirkstoffe | Wirkstoffkonzentration in g ai/ha | Wirkung in % nach 6[d] |
| 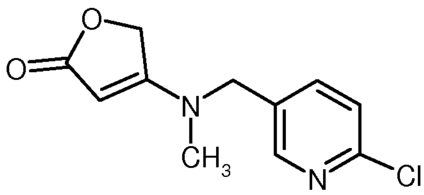 erfindungsgemäß (84) | 60 | 100 |
| 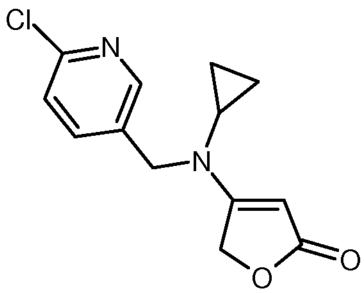 erfindungsgemäß (88) | 60 | 100 |

Beispiel F

**Aphis gossypii -Test** (Bodenapplikation)

Lösungsmittel: 4 Gewichtsteile Aceton
Emulgator: 1 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Die Wirkstoffzubereitung wird mit Erde vermischt. Die angegebene Konzentration bezieht sich auf die Wirkstoffmenge pro Volumeneinheit Boden (mg/l = ppm). Man füllt den behandelten Boden in Töpfe und bepflanzt ihn mit einer Baumwollpflanze *(Gossypium hirsutum)* bepflanzt. Nach einer Woche wird mit der Baumwollblattlaus (*Aphis gossypii*) infiziert.

Nach der gewünschten Zeit wird die Abtötung in % bestimmt. Dabei bedeutet 100 %, dass alle Blattläuse abgetötet wurden; 0 % bedeutet, dass keine Blattläuse abgetötet wurden.

Bei diesem Test zeigen z. B. die folgenden Verbindungen der Herstellungsbeispiele gute Wirksamkeit:

Tabelle F

| pflanzenschädigende Insekten **Aphis gossypii** (Bodenapplikation) | | |
|---|---|---|
| Wirkstoffe | Wirkstoffkonzentration in ppm | Wirkung in % nach 7[d] |
| 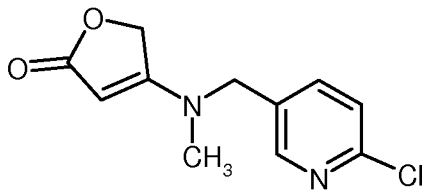 erfindungsgemäß (84) | 4 | 100 |

(fortgesetzt)

| pflanzenschädigende Insekten **Aphis gossypii** (Bodenapplikation) | | |
|---|---|---|
| Wirkstoffe | Wirkstoffkonzentration in ppm | Wirkung in % nach 7[d] |
| 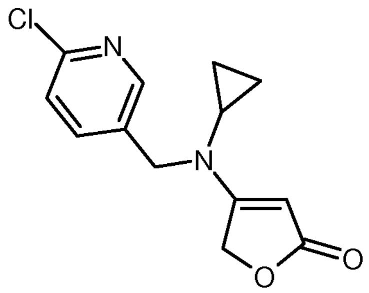 erfindungsgemäß (88) | 4 | 100 |

Beispiel G

**Myzus persicae -Test** (Bodenapplikation)

Lösungsmittel: 4 Gewichtsteile Aceton

Emulgator: 1 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Die Wirkstoffzubereitung wird mit Erde vermischt. Die angegebene Konzentration bezieht sich auf die Wirkstoffmenge pro Volumeneinheit Boden (mg/l = ppm). Man füllt den behandelten Boden in Töpfe und bepflanzt ihn mit einer Paprikapflanze (*Capsicum annuum*) Nach einer Woche wird mit der Grünen Pfirsichblattlaus *(Myzus persicae*) infiziert.

Nach der gewünschten Zeit wird die Abtötung in % bestimmt. Dabei bedeutet 100 %, dass alle Blattläuse abgetötet wurden; 0 % bedeutet, dass keine Blattläuse abgetötet wurden.

Bei diesem Test zeigen z. B. die folgenden Verbindungen der Herstellungsbeispiele gute Wirksamkeit:

Tabelle G

| pflanzenschädigende Insekten **Myzus persicae** (Bodenapplikation) | | |
|---|---|---|
| Wirkstoffe | Wirkstoffkonzentration in ppm | Wirkung in % nach 7[d] |
| 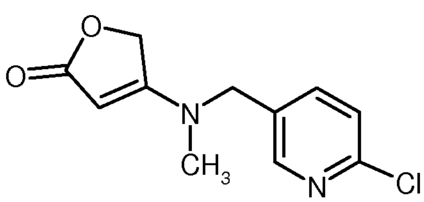 erfindungsgemäß (84) | 4 | 100 |

(fortgesetzt)

| pflanzenschädigende Insekten **Myzus persicae** (Bodenapplikation) | | |
|---|---|---|
| Wirkstoffe | Wirkstoffkonzentration in ppm | Wirkung in % nach 7d |
| 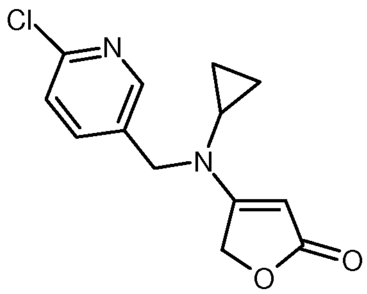 | | |
| erfindungsgemäß (88) | 4 | 100 |

Beispiel H

**Diabrotica balteata - Larven -Test** (Bodenapplikation)

Lösungsmittel: 4 Gewichtsteile Aceton
Emulgator: 1 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Die Wirkstoffzubereitung wird mit Erde vermischt. Die angegebene Konzentration bezieht sich auf die Wirkstoffmenge pro Volumeneinheit Boden (mg/l = ppm). Man füllt den behandelten Boden in Töpfe und legt je Topf 5 Maiskörner aus. 3 Tage nach Aussaat werden Larven des Maiswurzelbohrers (*Diabrotica balteata*) in den behandelten Boden gesetzt.

Nach der gewünschten Zeit wird die Abtötung in % bestimmt. Der Wirkungsgrad berechnet sich aus der Anzahl der aufgelaufenen Maispflanzen.

Bei diesem Test zeigt z. B. die folgende Verbindung der Herstellungsbeispiele gute Wirksamkeit:

Tabelle H

| pflanzenschädigende Insekten **Diabrotica balteata** (Bodenapplikation) | | |
|---|---|---|
| Wirkstoffe | Wirkstoffkonzentration in ppm | Wirkung in % nach 4d |
| 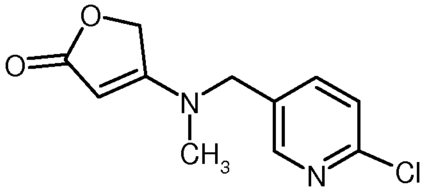 | | |
| erfindungsgemäß (84) | 8 | 100 |

(fortgesetzt)

| pflanzenschädigende Insekten **Diabrotica balteata** (Bodenapplikation) | | |
|---|---|---|
| Wirkstoffe | Wirkstoffkonzentration in ppm | Wirkung in % nach 4d |
| 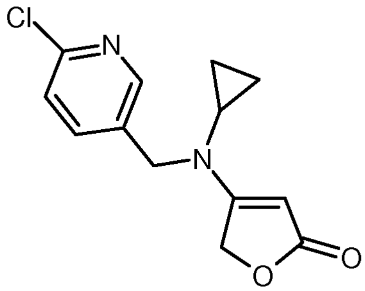 | | |
| erfindungsgemäß (88) | 8 | 90 |

Beispiel I

**Aphis gossypii -Test** (Saatgutapplikation)

| | | |
|---|---|---|
| Lösungsmittel: | 7 | Gewichtsteile Dimethylformamid |
| Emulgator: | 10 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Baumwollsaatgut *(Gossypium hirsutum)* wird mit der Wirkstoffzubereitung gebeizt und in Erde ausgesät. Nach ca. 2 Wochen werden die Baumwollpflanzen mit der Baumwollblattlaus (*Aphis gossypii*) infiziert.

Nach der gewünschten Zeit wird die Abtötung in % bestimmt. Dabei bedeutet 100 %, dass alle Blattläuse abgetötet wurden; 0 % bedeutet, dass keine Blattläuse abgetötet wurden.

Bei diesem Test zeigen z. B. die folgenden Verbindungen der Herstellungsbeispiele gute Wirksamkeit:

Tabelle I

| pflanzenschädigende Insekten **Aphis gossypii** (Saatgutapplikation) | | |
|---|---|---|
| Wirkstoffe | Wirkstoffkonzentration in g ai/kg | Wirkung in % nach 7d |
| 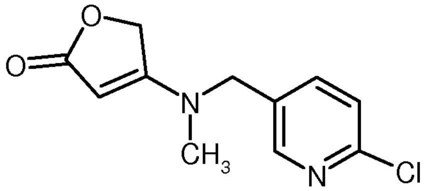 | | |
| erfindungsgemäß (84) | 4 | 100 |
| 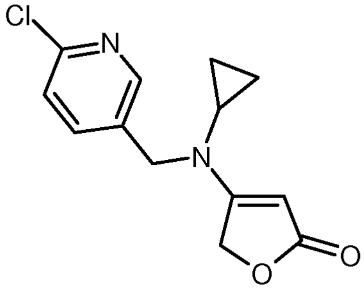 | | |
| erfindungsgemäß (88) | 4 | 100 |

Beispiel J

**Aphis fabae -Test** (Saatgutapplikation)

| | | |
|---|---|---|
| Lösungsmittel: | 7 | Gewichtsteile Dimethylformamid |
| Emulgator: | 10 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zuckerrübensaatgut (*Beta vulgaris*) wird mit der Wirkstoffzubereitung gebeizt und in Erde ausgesät. Nach ca. 4 Wochen werden die Rübenpflanzen mit der Schwarzen Bohnenblattlaus (*Aphis fabae*) infiziert.

Nach der gewünschten Zeit wird die Abtötung in % bestimmt. Dabei bedeutet 100 %, dass alle Blattläuse abgetötet wurden; 0 % bedeutet, dass keine Blattläuse abgetötet wurden.

Bei diesem Test zeigt z. B. die folgende Verbindung der Herstellungsbeispiele gute Wirksamkeit:

Tabelle J

pflanzenschädigende Insekten **Aphis fabae** (Saatgutapplikation)

| Wirkstoffe | Wirkstoffkonzentration in g ai/unit* | Wirkung in % nach 7$^d$ |
|---|---|---|
| 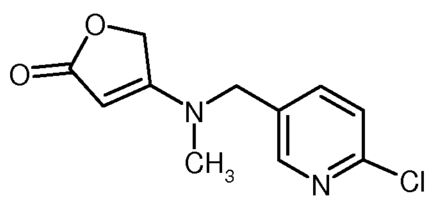 | | |
| erfindungsgemäß (84) | 90 | 100 |
| * 100.000 Körner | | |

Beispiel K

**Rhopalosiphon padi -Test** (Saatgutapplikation)

| | | |
|---|---|---|
| Lösungsmittel: | 7 | Gewichtsteile Dimethylformamid |
| Emulgator: | 10 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Gerstensaatgut (*Hordeum vulgare*) wird mit der Wirkstoffzubereitung gebeizt und in Erde ausgesät. Nach ca. 1 Woche werden die Gerstenpflanzen mit der Haferblattlaus (*Rhopalosiphon padi*) infiziert.

Nach der gewünschten Zeit wird die Abtötung in % bestimmt. Dabei bedeutet 100 %, dass alle Blattläuse abgetötet wurden; 0 % bedeutet, dass keine Blattläuse abgetötet wurden.

Bei diesem Test zeigen z. B. die folgenden Verbindungen der Herstellungsbeispiele gute Wirksamkeit:

| Tabelle K<br>pflanzenschädigende Insekten **Rhopalosiphon padi** (Saatgutapplikation) | | |
|---|---|---|
| Wirkstoffe | Wirkstoffkonzentration in g ai/kg | Wirkung in % nach 7$^{d}$ |
| 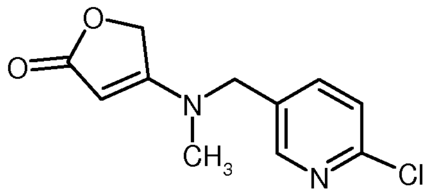<br>erfindungsgemäß (84) | 1,4 | 100 |
| 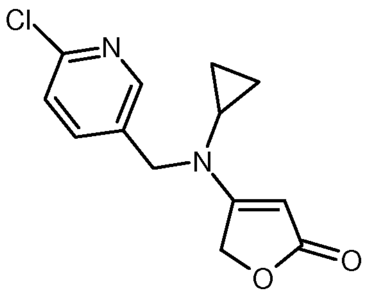<br>erfindungsgemäß (88) | 1,4 | 100 |

Beispiel L

**Myzus-Test** (Spritzbehandlung)

| | | |
|---|---|---|
| Lösungsmittel: | 78 | Gewichtsteile Aceton |
| | 1,5 | Gewichtsteile Dimethylformamid |
| Emulgator: | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Chinakohlblattscheiben (*Brassica pekinensis*), die von allen Stadien der Grünen Pfirsichblattlaus (*Myzus persicae*) befallen sind, werden mit einer Wirkstoffzubereitung der gewünschten Konzentration gespritzt.

Nach der gewünschten Zeit wird die Wirkung in % bestimmt. Dabei bedeutet 100 %, dass alle Blattläuse abgetötet wurden; 0 % bedeutet, dass keine Blattläuse abgetötet wurden.

Bei diesem Test zeigen z. B. die folgenden Verbindungen der Herstellungsbeispiele gute Wirksamkeit:

| Tabelle L Blatt 1<br>pflanzenschädigende Insekten **Myzus -Test** (Spritzbehandlung) | | |
|---|---|---|
| Wirkstoffe | Wirkstoffkonzentration in g/ha | Abtötungsgrad in % nach 5$^{d}$ |
| 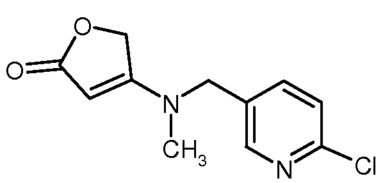<br>erfindungsgemäß (84) | 500 | 100 |

(fortgesetzt)

pflanzenschädigende Insekten **Myzus -Test** (Spritzbehandlung)

| Wirkstoffe | Wirkstoffkonzentration in g/ha | Abtötungsgrad in % nach 5[d] |
|---|---|---|
| 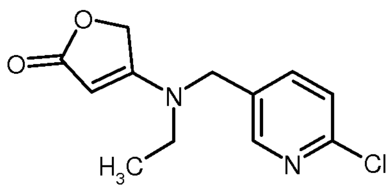 | | |
| erfindungsgemäß (85) | 500 | 90 |
| 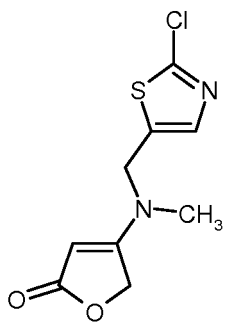 | | |
| erfindungsgemäß (144) | 500 | 100 |

Tabelle L Blatt 2

pflanzenschädigende Insekten **Myzus -Test** (Spritzbehandlung)

| Wirkstoffe | Wirkstoffkonzentration in g/ha | Abtötungsgrad in % nach 5[d] |
|---|---|---|
| 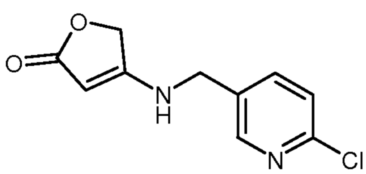 | | |
| erfindungsgemäß (83) | 500 | 90 |
| 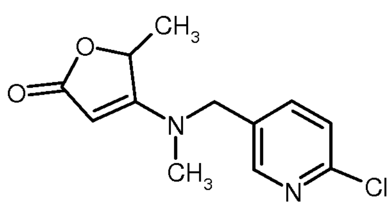 | | |
| erfindungsgemäß (117) | 500 | 100 |

(fortgesetzt)

pflanzenschädigende Insekten **Myzus -Test** (Spritzbehandlung)

| Wirkstoffe | Wirkstoffkonzentration in g/ha | Abtötungsgrad in % nach 5[d] |
|---|---|---|
| 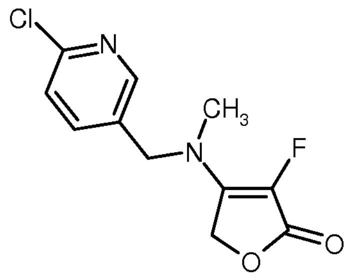 erfindungsgemäß (104) | 500 | 100 |

Tabelle L Blatt 3

pflanzenschädigende Insekten **Myzus -Test** (Spritzbehandlung)

| Wirkstoffe | Wirkstoffkonzentration in g/ha | Abtötungsgrad in % nach 5[d] |
|---|---|---|
| 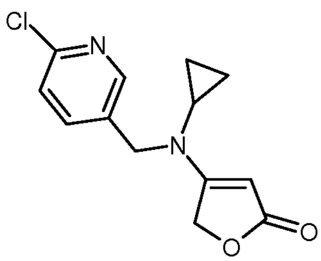 erfindungsgemäß (88) | 500 | 100 |
| 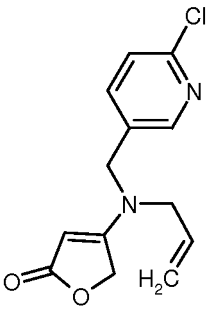 erfindungsgemäß (91) | 500 | 100 |
| 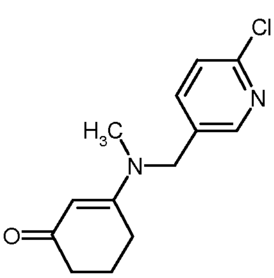 erfindungsgemäß (2) | 500 | 90 |

Tabelle L Blatt 4

pflanzenschädigende Insekten **Myzus -Test** (Spritzbehandlung)

| Wirkstoffe | Wirkstoffkonzentration in g/ha | Abtötungsgrad in % nach 5[d] |
|---|---|---|
| 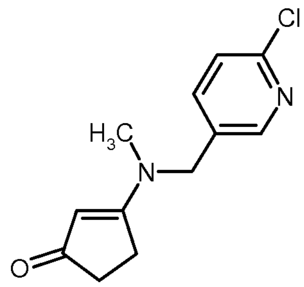 erfindungsgemäß (175) | 500 | 100 |
| 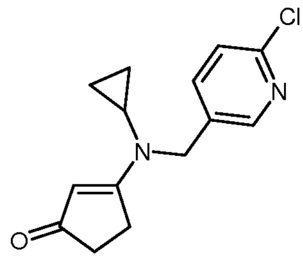 erfindungsgemäß (179) | 500 | 100 |
| 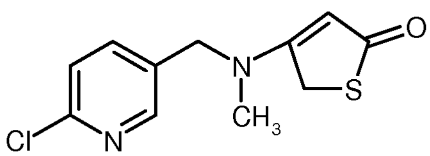 erfindungsgemäß (112) | 500 | 80 |

Bei einer Aufwandmenge von jeweils 500g/ha zeigten die Verbindungen gemäß den Beispielen 1, 98, 94, 147, 125, 121, 4, 9, 10, 124, 126, 92 und 70 nach 5 Tagen jeweils 100 % Wirkung, die Verbindung gemäß Beispiel 3 90 % Wirkung.

Beispiel M

**Phaedon cochleariae -Test** (Spritzbehandlung)

| | | |
|---|---|---|
| Lösungsmittel: | 78 | Gewichtsteile Aceton |
| | 1,5 | Gewichtsteile Dimethylformamid |
| Emulgator: | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Chinakohlblattscheiben *(Brassica pekinensis)* werden mit einer Wirkstoffzubereitung der gewünschten Konzentration gespritzt und nach dem Abtrocknen mit Larven des Meerrettichblattkäfers *(Phaedon cochleariae)* besetzt.

Nach der gewünschten Zeit wird die Wirkung in % bestimmt. Dabei bedeutet 100 %, dass alle Käferlarven abgetötet wurden; 0 % bedeutet, dass keine Käferlarven abgetötet wurden.

Bei diesem Test zeigen z. B. die folgenden Verbindungen der Herstellungsbeispiele gute Wirksamkeit:

Tabelle M Blatt 1

pflanzenschädigende Insekten **Phaedon cochleariae -Test** (Spritzbehandlung)

| Wirkstoffe | Wirkstoffkonzentration in g/ha | Abtötungsgrad in % nach $7^d$ |
|---|---|---|
| 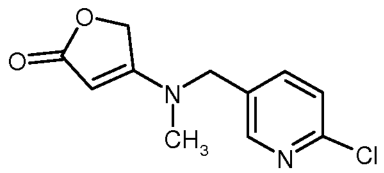<br>erfindungsgemäß (84) | 500 | 100 |
| 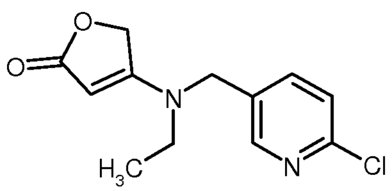<br>erfindungsgemäß (85) | 500 | 100 |
| 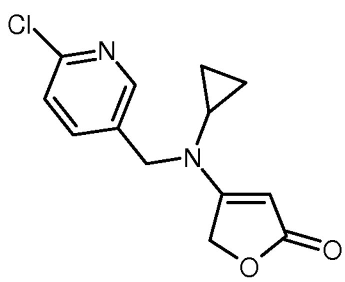<br>erfindungsgemäß (88) | 500 | 100 |

Tabelle M Blatt 2

pflanzenschädigende Insekten **Phaedon cochleariae -Test** (Spritzbehandlung)

| Wirkstoffe | Wirkstoffkonzentration in g/ha | Abtötungsgrad in % nach $7^d$ |
|---|---|---|
| 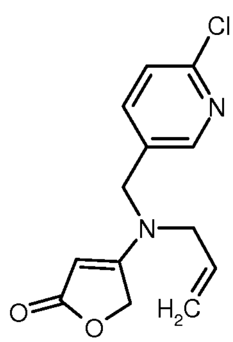<br>erfindungsgemäß (91) | 500 | 67 |

Bei einer Aufwandmenge von jeweils 500g/ha zeigten die Verbindungen gemäß den Beispielen 98 und 121 nach 7 Tagen eine Wirkung von jeweils 100 %.

Beispiel N

**Spodoptera frugiperda-Test** (Spritzbehandlung)

| | | |
|---|---|---|
| Lösungsmittel: | 78 | Gewichtsteile Aceton |
| | 1,5 | Gewichtsteile Dimethylformamid |
| Emulgator: | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Maisblattscheiben (*Zea mays*) werden mit einer Wirkstoffzubereitung der gewünschten Konzentration gespritzt und nach dem Abtrocknen mit Raupen des Heerwurms (*Spodoptera frugiperda*) besetzt.

Nach der gewünschten Zeit wird die Wirkung in % bestimmt. Dabei bedeutet 100 %, dass alle Raupen abgetötet wurden; 0 % bedeutet, dass keine Raupen abgetötet wurden.

Bei diesem Test zeigen z. B. die folgenden Verbindungen der Herstellungsbeispiele gute Wirksamkeit:

Tabelle N

pflanzenschädigende Insekten **Spodoptera frugiperda -Test** (Spritzbehandlung)

| Wirkstoffe | Wirkstoffkonzentration in g/ha | Abtötungsgrad in % nach 7[d] |
|---|---|---|
| 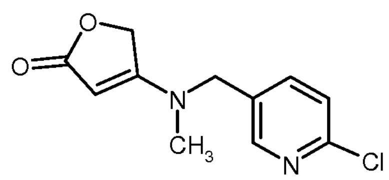 | | |
| erfindungsgemäß (84) | 500 | 100 |
| 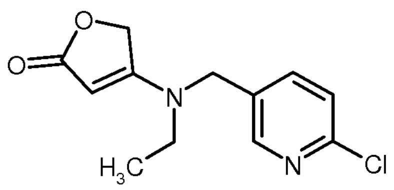 | | |
| erfindungsgemäß (85) | 500 | 100 |
| 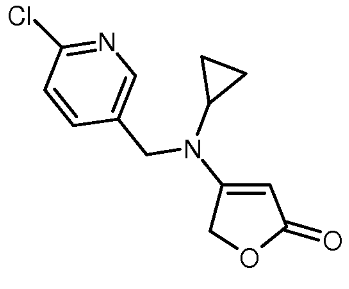 | | |
| erfindungsgemäß (88) | 500 | 100 |

Bei einer Aufwandmenge von jeweils 500g/ha zeigte auch die Verbindung gemäß Beispiel 121 nach 7 Tagen eine Wirkung von 100 %.

Beispiel **O**

**Heliothis virescens-Test** (Spritzbehandlung)

| | | |
|---|---|---|
| Lösungsmittel: | 78 | Gewichtsteile Aceton |
| | 1,5 | Gewichtsteile Dimethylformamid |
| Emulgator: | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Sojabohnenblätter *(Glycine max.)* werden mit einer Wirkstoffzubereitung der gewünschten Konzentration gespritzt und nach dem Abtrocknen mit Eiern des Baumwollkapselwurms *(Heliotis virescens)* besetzt.

Nach der gewünschten Zeit wird die Wirkung in % bestimmt. Dabei bedeutet 100 %, dass alle Eier abgetötet wurden; 0 % bedeutet, dass keine Eier abgetötet wurden.

Bei diesem Test zeigen z. B. die folgenden Verbindungen der Herstellungsbeispiele gute Wirksamkeit:

Tabelle O

pflanzenschädigende Insekten **Heliothis virescens -Test** (Spritzbehandlung)

| Wirkstoffe | Wirkstoffkonzentration in g/ha | Abtötungsgrad in % nach 7$^d$ |
|---|---|---|
| 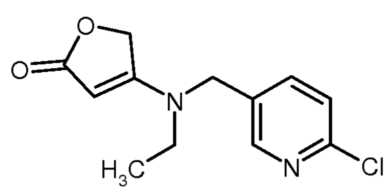 | | |
| erfindungsgemäß (85) | 500 | 100 |
| 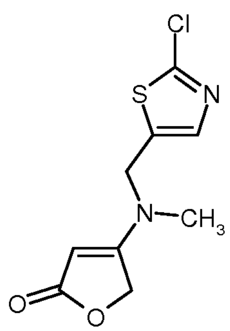 | | |
| erfindungsgemäß (144) | 500 | 99 |

Beispiel P

**Aphis gossypii -Test**

| | | |
|---|---|---|
| Lösungsmittel: | 7 | Gewichtsteile Dimethylformamid |
| Emulgator: | 2 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Baumwollblätter (*Gossypium hirsutum*), die stark von der Baumwollblattlaus (*Aphis gossypii*) befallen sind, werden durch Tauchen in die Wirkstoffzubereitung der gewünschten Konzentration behandelt.

Nach der gewünschten Zeit wird die Abtötung in % bestimmt. Dabei bedeutet 100 %, dass alle Blattläuse abgetötet wurden; 0 % bedeutet, dass keine Blattläuse abgetötet wurden.

Bei diesem Test zeigt z. B. die folgende Verbindung der Herstellungsbeispiele gute Wirksamkeit:

Tabelle P Blatt 1

pflanzenschädigende Insekten **Aphis gossypii -Test**

| Wirkstoffe | Wirkstoffkonzentration in g/ha | Abtötungsgrad in % nach 6$^{d}$ |
|---|---|---|
| 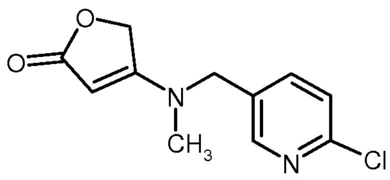 | | |
| erfindungsgemäß (84) | 100 | 100 |
| 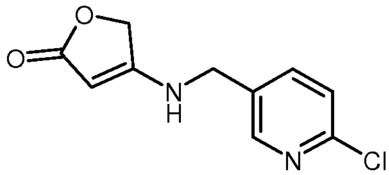 | | |
| erfindungsgemäß (83) | 100 | 98 |
| 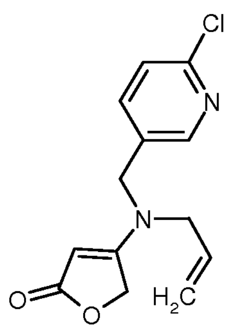 | | |
| erfindungsgemäß (91) | 100 | 95 |

Tabelle P Blatt 2

pflanzenschädigende Insekten **Aphis gossypii -Test**

| Wirkstoffe | Wirkstoffkonzentration in g/ha | Abtötungsgrad in % nach 6$^{d}$ |
|---|---|---|
| 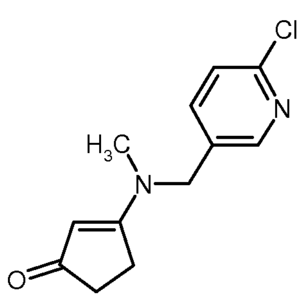 | | |

(fortgesetzt)

pflanzenschädigende Insekten **Aphis gossypii -Test**

| Wirkstoffe | Wirkstoffkonzentration in g/ha | Abtötungsgrad in % nach $6^d$ |
|---|---|---|
| erfindungsgemäß (175) | 100 | 95 |

Bei einer Wirkstoffkonzentration von jeweils 100 ppm zeigten die Verbindungern gemäß den Beispielen 98 und 121 nach 6 Tagen eine Wirkung von jeweils 100 %, die Verbindung gemäß Beispiel 125 eine Wirkung von 95 %.

Bei einer Wirkstoffkonzentration von 20 ppm zeigte die Verbindung gemäß Beispiel 85 nach 6 Tagen eine Wirkung von 97 %.

Beispiel Q

**Plutella-Test**

| | | |
|---|---|---|
| Lösungsmittel: | 100 | Gewichtsteile Aceton |
| | 1900 | Gewichtsteile Methanol |

Zur Herstellung einer zweckmäßigen Wirkstoffzubereitung vermischt man 1 Gewichtsteil Wirkstoff mit der angegebenen Menge Lösungsmittel und verdünnt das Konzentrat mit Methanol auf die gewünschten Konzentrationen.

Auf eine genormte Menge Kunstfutter wird eine angegebene Menge Wirkstoffzubereitung der gewünschten Konzentration pipettiert. Nachdem das Methanol verdunstet ist, werden ca. 200-300 Eier der Kohlschabe (*Plutella xylostella*) auf das Futter gegeben.

Nach der gewünschten Zeit wird die Abtötung der Eier bzw. Larven in % bestimmt. Dabei bedeutet 100%, daß alle Tiere abgetötet wurden; 0% bedeutet, daß keine Tiere abgetötet wurden.

Bei diesem Test zeigt z. B. die folgende Verbindung der Herstellungsbeispiele gute Wirksamkeit:

Tabelle Q

pflanzenschädigende Insekten **Plutella-Test**

| Wirkstoffe | Wirkstoffkon- zentration in g/ha | Abtötungsgrad in % nach $7^d$ |
|---|---|---|
| 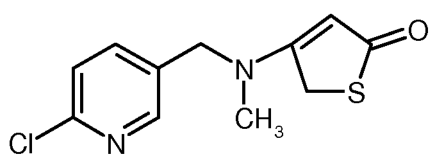 | | |
| erfindungsgemäß (112) | 1000 | 100 |

Beispiel R

**Myzus persicae -Test** (hydroponische Behandlung)

| | | |
|---|---|---|
| Lösungsmittel: | 7 | Gewichtsteile Dimethylformamid |
| Emulgator: | 2 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Die Wirkstoffzubereitung wird mit Wasser gemischt. Die angegebene Konzentration bezieht sich auf die Wirkstoffmenge pro Volumeneinheit Wasser (mg/l = ppm). Man füllt das behandelte Wasser in Gefäße mit einer Erbsenpflanze (*Pisum sativum*). Nach der vorgegebenen Zeit wird mit der Grünen Pfirsichblattlaus (*Myzus persicae*) infiziert.

Nach der gewünschten Zeit wird die Abtötung in % bestimmt. Dabei bedeutet 100 %, dass alle Blattläuse

abgetötet wurden; 0 % bedeutet, dass keine Blattläuse abgetötet wurden.

Bei diesem Test zeigt z. B. die folgende Verbindung der Herstellungsbeispiele gute Wirksamkeit:

Tabelle R

pflanzenschädigende Insekten **Myzus persicae-Test** (hydroponische Behandlung)

| Wirkstoffe | Wirkstoffkonzentration in g/ha | Abtötungsgrad in % nach $7^d$ |
|---|---|---|
| 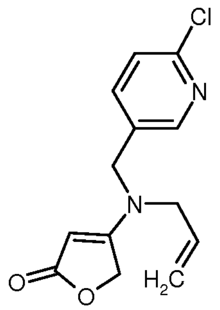 | | |
| erfindungsgemäß (91) | 20 | 100 |

Bei einer Wirkstoffkonzentration von jeweils 20 ppm zeigten die Verbindungern gemäß den Beispielen 98, 121 und 125 nach 7 Tagen eine Wirkung von jeweils 100 %.

Beispiel S

**Nilaparvata lugens-Test; hydroponische Behandlung** (NILALU SYS)

| | | |
|---|---|---|
| Lösungsmittel: | 78 | Gewichtsteile Aceton |
| | 1,5 | Gewichtsteile Dimethylformamid |
| Emulgator: | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Die Wirkstoffzubereitung wird in Wasser pipettiert. Die angegebene Konzentration bezieht sich auf die Wirkstoffmenge pro Volumeneinheit Wasser (mg/l = ppm), anschließend wird mit der Braunrückigen Reiszikade (*Nilaparvata lugens*) infiziert.

Nach der gewünschten Zeit wird die Wirkung in % bestimmt. Dabei bedeutet 100 %, dass alle Reiszikaden abgetötet wurden; 0 % bedeutet, dass keine Reiszikaden abgetötet wurden.

Bei diesem Test zeigte die Verbindung gemäß Beispiel 1 bei einer Aufwandmenge von 500 g/ha nach 7 Tagen eine Wirkung von 100 %.

Beispiel T

**Test mit Katzenflöhen / orale Aufnahme**

| | |
|---|---|
| Testtiere: | Adulte *Ctenocephalides felis* |
| Lösungsmittel: | Dimethylsulfoxid (DMSO) |

Zwecks Herstellung einer geeigneten Formulierung wird aus 10 mg Wirkstoff mit 0,5 ml DMSO eine geeignete Wirkstofflösung hergestellt. 10 µl dieser Formulierung werden zu 2 ml citriertem Rinderblut gegeben und verrührt.

20 nüchterne adulte Flöhe (*Ctenocephalides felis,* Stamm "Georgi") werden in eine Kammer (∅ 5 cm) eingesetzt, die oben und unten mit Gaze verschlossen ist. Auf die Kammer wird ein Metallzylinder gestellt, dessen Unterseite mit Parafilm verschlossen ist. Der Zylinder enthält die 2 ml Blut-Wirkstofformulierung, die von den Flöhen durch die

Parafilmmembran aufgenommen werden kann. Während das Blut auf 37° C erwärmt wird, ist im Bereich der Flohkammern Raumtemperatur. Kontrollen werden mit dem gleichen Volumen DMSO ohne Zusatz einer Verbindung vermischt.

Nach der gewünschten Zeit wird die Abtötung in % bestimmt. Dabei bedeutet 100 %, dass alle Flöhe abgetötet wurden; 0 % bedeutet, dass kein Floh abgetötet wurde.

Bei einer Wirkstoffkonzentration von 100 ppm bewirkte die Verbindung gemäß Beispiel 84 nach 2 Tagen eine Abtötung von 100 %.

Beispiel U

**Fliegenlarven-Test**

| | |
|---|---|
| Testtiere: | *Lucilia cuprina*-Larven |
| Lösungsmittel: | Dimethylsulfoxid |

10 mg Wirkstoff werden in 0,5 ml Dimethylsulfoxid gelöst. Zwecks Herstellung einer geeigneten Formulierung verdünnt man die Wirkstofflösung mit Wasser auf die jeweils gewünschte Konzentration.

Etwa 20 *Lucilia cuprina*-Larven werden in ein Teströhrchen gebracht, welches ca. 1 $cm^3$ Pferdefleisch und 0.5 ml der zu testende Wirkstoffzubereitung enthält.

Nach der gewünschten Zeit wird die Abtötung in % bestimmt. Dabei bedeutet 100 %, dass alle Larven abgetötet wurden; 0 % bedeutet, dass keine Larve abgetötet wurde.

Bei einer Wirkstoffkonzentration von jeweils 100 ppm bewirkten die Verbindungen gemäß den Beispielen 98, 121, 84, 85, 88, 104 und 175 nach 2 Tagen eine Abtötung von 100 %, die Verbindung gemäß Beispiel 125 von 90 %.

Beispiel V

**Test mit Fliegen**

| | |
|---|---|
| Testtiere: | adulte *Musca domestica,* Stamm WHO(N), sensibel |
| Lösungsmittel: | Dimethylsulfoxid |

10 mg Wirkstoff werden in 0,5 ml Dimethylsulfoxid gelöst, geringere Konzentrationen werden durch verdünnen mit Wasser hergestellt. 0,2 ml dieser Wirkstoffzubereitung werden auf einen Schwam (ca. ∅ 1,5 cm), der mit 0,8 ml Zuckerlösung getränkt ist, pipettiert. Der Schwam und 10 Testtiere werden in eine Schale (4x4 cm, H 2 cm) überführt und abgedeckt.

Nach 48 Stunden wird die Wirksamkeit der Wirkstoffzubereitung ermittelt. Dabei bedeutet 100 %, dass alle Fliegen abgetötet wurden; 0 % bedeutet, dass keine Fliegen abgetötet wurden.

Bei einer Wirkstoffkonzentration von jeweils 100 ppm zeigte die Verbindung gemäß Beispiel 84 eine Wirkung von 80 %.

Beispiel W

**Test mit resistenten einwirtigen Rinderzecken/SP-resistenter Parkhurst-Stamm**

**Injektionsverfahren** *Boophilus microplus (INJ)*

| | |
|---|---|
| Testtiere: | adulte gesogene Weibchen von *Boophilus microplus* (Stamm Parkhurst - SP-resistent) |
| Lösungsmittel: | Dimethylsulfoxid |

10 mg Wirkstoff werden in 0,5 ml Dimethylsulfoxid gelöst, geringere Konzentrationen werden durch verdünnen in dem gleichen Lösungsmittel hergestellt.

Der Test wird in 5-fach-Bestimmung durchgeführt. 1 µl der Lösungen wird in das Abdomen injiziert, die Tiere in Schalen überführt und in einem klimatisierten Raum aufbewahrt. Die Wirkungungskontrolle erfolgt nach 7 Tagen auf Ablage fertiler Eier. Eier deren Fertilität nicht äußerlich sichtbar ist, werden in Glasröhrchen bis zum Larvenschlupf nach etwa 24 Tagen im Klimaschrank aufbewahrt. Eine Wirkung von 100 % bedeutet, daß keine Zecke fertile Eier gelegt hat.
Bei einer Aufwandmenge von jeweils 20 µg/Tier zeigte die Verbindung gemäß Beispiel 125 eine Wirkung von 80 %, die Verbindung gemäß Beispiel 121 eine Wirkung von 95 %.

Beispiel X

**Cydia pomonella -Test**

| | | |
|---|---|---|
| Lösungsmittel: | 4 | Gewichtsteile Aceton |
| Emulgator: | 1 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.
Die Wirkstoffzubereitung wird mit Futter vermischt. Die angegebene Konzentration bezieht sich auf die Wirkstoffmenge pro Volumeneinheit Futter (mg/l = ppm). Man füllt das behandelte Futter in Petrischalen und infiziert es mit Apfelwicklerlarven *(Cydia pomonella).*
Nach der gewünschten Zeit wird die Abtötung in % bestimmt. Dabei bedeutet 100 %, dass alle Larven abgetötet wurden; 0 % bedeutet, dass keine Larven abgetötet wurden.
Bei einer Wirkstoffkonzentration von 20 ppm bewirkte die Verbindung gemäß Beispiel 84 nach 7 Tagen eine Abtötung von 100 %, die Verbindung gemäß Beispiel 88 eine Abtötung von 90 %.

Beispiel Y

**Leptinotarsa decemlineata - Adulte -Test**

| | | |
|---|---|---|
| Lösungsmittel: | 7 | Gewichtsteile Dimethylformamid |
| Emulgator: | 10 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.
Blätter von Kartoffelpflanzen (*Solanum tuberosum*), die mit Adulten des Kartoffelkäfern (*Leptinotarsa decemlineata*) besetzt sind, werden durch Spritzen mit der Wirkstoffzubereitung in der gewünschten Konzentration behandelt.
Nach der gewünschten Zeit wird die Wirkung in % bestimmt. Dabei bedeutet 100 %, dass alle Käfer abgetötet wurden; 0 % bedeutet, dass keine Käfer abgetötet wurden.
Bei einer Aufwandmenge von jeweils 60 g/ha zeigten die Verbindungen gemäß den Beispielen 84 und 88 nach 7 Tagen eine Wirkung von jeweils 100 %.

Beispiel Z

**Bemisia tabaci -Test**

| | | |
|---|---|---|
| Lösungsmittel: | 4 | Gewichtsteile Aceton |
| Emulgator: | 1 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.
Die Wirkstoffzubereitung wird mit Erde vermischt. Die angegebene Konzentration bezieht sich auf die Wirkstoffmenge pro Volumeneinheit Boden (mg/l = ppm). Man füllt den behandelten Boden in Töpfe und bepflanzt ihn mit einer Baumwollpflanze (*Gossypium hirsutum*) bepflanzt. Nach einer Woche wird mit der Weißen Fliege (*Bemisia tabaci*) zur Eiablage infiziert.

Nach der gewünschten Zeit wird die Abtötung der Eier bzw. Larven in % bestimmt. Dabei bedeutet 100 %, dass alle Tiere abgetötet wurden; 0 % bedeutet, dass keine Tiere abgetötet wurden.

Bei einer Wirkstoffkonzentration von jeweils 4 ppm bewirkten die Verbindungen gemäß den Beispielen 84 und 88 nach 14 Tage eine Abtötung von jeweils 100 %.

Beispiel A1

**Spodoptera frugiperda -Test**

| | | |
|---|---|---|
| Lösungsmittel: | 4 | Gewichtsteile Aceton |
| Emulgator: | 1 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Die Wirkstoffzubereitung wird mit Erde vermischt. Die angegebene Konzentration bezieht sich auf die Wirkstoffmenge pro Volumeneinheit Boden (mg/l = ppm). Man füllt den behandelten Boden in Töpfe und bepflanzt ihn mit einer Baumwollpflanze (*Gossypium hirsutum*) bepflanzt. Nach einer Woche wird mit Raupen des Heerwurms (*Spodoptera frugiperda*) infiziert.

Nach der gewünschten Zeit wird die Wirkung in % bestimmt. Dabei bedeutet 100 %, dass kein Fraßschaden sichtbar ist; 0 % bedeutet, dass der Fraßschaden an den behandelten Planzen der der Kontrolle entspricht..

Bei einer Wirkstoffkonzentration von 4 ppm zeigte die Verbindung gemäß Beispiel 84 eine Wirkung von 98 % (nach 7 Tagen).

Beispiel B1

**Pulvinaria regalis -Test**

| | | |
|---|---|---|
| Lösungsmittel: | 7 | Gewichtsteile Dimethylformamid |
| Emulgator: | 10 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Kastanienbäume *(Castaney vesca)*, die von der Wolligen Napfschildlaus (*Pulvinaria regalis*) befallen sind, werden durch Sprühen mit der Wirkstoffzubereitung in der gewünschten Konzentration behandelt.

Nach der gewünschten Zeit wird die Abtötung in % bestimmt. Dabei bedeutet 100 %, dass alle Tiere abgetötet wurden; 0 % bedeutet, dass keine Tiere abgetötet wurden.

Bei einer Wirkstoffkonzentration von jeweils 100 ppm bewirkte die Verbindung gemäß Beispiel 84 nach 30 Tagen eine Abtötung von 100 %, die Verbindung gemäß Beispiel 88 eine Abtötung von 95 %.

Beispiel C1

**Pulvinaria regalis -Test**

| | | |
|---|---|---|
| Lösungsmittel: | 4 | Gewichtsteile Aceton |
| Emulgator: | 1 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte

Konzentration.

Die Wirkstoffzubereitung wird an Kastanienbäume *(Castanea vesca)* angegossen. Die angegebene Konzentration bezieht sich auf die Wirkstoffmenge pro Pflanze. Nach einer definierten Zeit wird mit der Wolligen Napfschildlaus *(Pulvinaria regalis)* infiziert.

Nach der gewünschten Zeit wird die Abtötung in % bestimmt. Dabei bedeutet 100 %, dass alle Tiere abgetötet wurden; 0 % bedeutet, dass keine Tiere abgetötet wurden.

Bei einer Aufwandmenge von 10 mg Wirkstoff pro Pflanze bewirkte die Verbindung gemäß Beispiel 84 nach 30 Tagen eine Abtötung von 100 %.

<u>Beispiel D1</u>

**Aphis fabae -Test** (Saatgutapplikation)

| | | |
|---|---|---|
| Lösungsmittel: | 7 | Gewichtsteile Dimethylformamid |
| Emulgator: | 10 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zuckerrübensaatgut (*Beta vulgaris*) wird mit der Wirkstoffzubereitung gebeizt und in Erde ausgesät. Nach ca. 4 Wochen werden die Rübenpflanzen mit der Schwarzen Bohnenblattlaus (*Aphis fabae*) infiziert.

Nach der gewünschten Zeit wird die Abtötung in % bestimmt. Dabei bedeutet 100 %, dass alle Blattläuse abgetötet wurden; 0 % bedeutet, dass keine Blattläuse abgetötet wurden.

Bei einer Aufwandmenge von 90 g pro Einheit (100.000 Körner) bewirkte die Verbindung gemäß Beispiel 88 nach 7 Tage eine Abtötung von 100 %.

<u>Beispiel E1</u>

**Odontotermes - Test; Freiland (Spritzbehandlung)**

Zur Herstellung einer zweckmäßigen Wirkstoffzubereitung vermischt man 1 Gewichtsteil Wirkstoff mit den angegebenen Mengen Wasser auf die gewünschte Konzentration.

Holzstücke des Sambabaumes *(Triplochiton scleroxylon)* werden durch Spritzen mit der Wirkstoffzubereitung der gewünschten Konzentration behandelt und mit Termiten *(Odontotermes sp.)* besetzt.

Nach der gewünschten Zeit wird die Wirkung in % bestimmt. Dabei bedeutet 100 %, das Holz wurde nicht von Termiten befallen; 0 % bedeutet, dass Holz wurde von Termiten befallen.

Bei einer Aufwandmenge von 240 g/ha zeigte die Verbindung gemäß Beispiel 84 nach 45 Tagen eine Wirkung von 99 %, die Verbindung gemäß Beispiel 88 nach 65 Tagen eine Wirkung von 95 %.

<u>Beispiel F1</u>

**Bemisia tabaci - Test; Freiland (Spritzbehandlung)**

Zur Herstellung einer zweckmäßigen Wirkstoffzubereitung vermischt man 1 Gewichtsteil Wirkstoff mit den angegebenen Mengen Wasser auf die gewünschte Konzentration.

Paparikapflanzen *(Cápsicum áinnuum),* die von allen Stadien der Weißen Fliege *(Bemisia tabaci)* befallen sind werden mit einer Wirkstoffzubereitung der gewünschten Konzentration gespritzt.

Nach der gewünschten Zeit wird die Abtötung in % bestimmt. Dabei bedeutet 100 %, dass alle Weiße Fliegen abgetötet wurden; 0 % bedeutet, dass keine Weiße Fliege abgetötet wurde.

Bei einer Aufwandmenge von 300 g/ha bewirkten die Verbindungen gemäß den Beispielen 84 und 88 nach 65 Tagen eine Abtötung von jeweils 93 %.

Beispiel G1

**Thrips tabaci - Test; Freiland (Spritzbehandlung)**

Zur Herstellung einer zweckmäßigen Wirkstoffzubereitung vermischt man 1 Gewichtsteil Wirkstoff mit den angegebenen Mengen Wasser auf die gewünschte Konzentration.
Zwiebeln *(Allium cépa),* die von allen Stadien des Tabaktripses *(Thrips tabaci)* befallen sind werden mit einer Wirkstoffzubereitung der gewünschten Konzentration gespritzt.
Nach der gewünschten Zeit wird die Abtötung in % bestimmt. Dabei bedeutet 100 %, dass alle Thripse abgetötet wurden; 0 % bedeutet, dass keine Thripse abgetötet wurde.
Bei einer Aufwandmenge von 300 g/ha bewirkte die Verbindung gemäß Beispiel 84 nach 14 Tagen eine Abtötung von 89 %, die Verbindung gemäß Beispiel 88 eine Abtötung von 98 %.

Beispiel H1

**Piezodorus guildingi - Test; Freiland (Spritzbehandlung)**

Zur Herstellung einer zweckmäßigen Wirkstoffzubereitung vermischt man 1 Gewichtsteil Wirkstoff mit den angegebenen Mengen Wasser auf die gewünschte Konzentration.
Sojabohnenpflanzen *(Glycine max),* die mit Adulten der Grünen Sojastinkwanze *(Piezodorus guildingi)* besetzt sind werden mit einer Wirkstoffzubereitung der gewünschten Konzentration gespritzt.
Nach der gewünschten Zeit wird die Abtötung in % bestimmt. Dabei bedeutet 100 %, dass alle Wanzen abgetötet wurden; 0 % bedeutet, dass keine Wanze abgetötet wurde.
Bei einer Aufwandmenge von 300 g/ha bewirkte die Verbindung gemäß Beispiel 84 nach 11 Tagen eine Abtötung von 100 %.

Beispiel I1

**Nilaparvata lugens - Test; Freiland** (Spritzbehandlung)

Zur Herstellung einer zweckmäßigen Wirkstoffzubereitung vermischt man 1 Gewichtsteil Wirkstoff mit den angegebenen Mengen Wasser auf die gewünschte Konzentration.
Reispflanzen *(Oryza sativa),* die von allen Stadien der Braunrückigen Reiszikade *(Nilaparvata lugens)* befallen sind werden mit einer Wirkstoffzubereitung der gewünschten Konzentration gespritzt.
Nach der gewünschten Zeit wird die Abtötung in % bestimmt. Dabei bedeutet 100 %, dass alle Zikaden abgetötet wurden; 0 % bedeutet, dass keine Zikade abgetötet wurde.
Bei einer Aufwandmenge von 300 g/ha bewirkte die Verbindung gemäß Beispiel 84 nach 14 Tagen eine Abtötung von jeweils 91 %, die Verbindung gemäß Beispiel 88 eine Abtötung von 99 %.

Beispiel J1

**Brevicoryne brassicae - Test; Freiland** (Spritzbehandlung)

Zur Herstellung einer zweckmäßigen Wirkstoffzubereitung vermischt man 1 Gewichtsteil Wirkstoff mit den angegebenen Mengen Wasser auf die gewünschte Konzentration.
Kohlpflanzen *(Brassica oleracea),* die von allen Stadien der Mehligen Kohlblattlaus *(Brevicoryne brassicae)* befallen sind werden mit einer Wirkstoffzubereitung der gewünschten Konzentration gespritzt.
Nach der gewünschten Zeit wird die Abtötung in % bestimmt. Dabei bedeutet 100 %, dass alle Blattläuse abgetötet wurden; 0 % bedeutet, dass keine Blattlaus abgetötet wurde.
Bei einer Aufwandmenge von 300 g/ha bewirkten die Verbindungen gemäß den Beispielen 84 und 88 nach 22 Tagen eine Abtötung von jeweils 99 %.

Beispiel K1

**Nephotettix sp. - Test; Freiland** (Spritzbehandlung)

Zur Herstellung einer zweckmäßigen Wirkstoffzubereitung vermischt man 1 Gewichtsteil Wirkstoff mit den angegebenen Mengen Wasser auf die gewünschte Konzentration.

Reispflanzen *(Oryza sativa),* die von allen Stadien der Grünen Reiszikade *(Nephotettix sp.)* befallen sind werden mit einer Wirkstoffzubereitung der gewünschten Konzentration gespritzt.
Nach der gewünschten Zeit wird die Abtötung in % bestimmt. Dabei bedeutet 100 %, dass alle Zikaden abgetötet wurden; 0 % bedeutet, dass keine Zikade abgetötet wurde.
Bei einer Aufwandmenge von 300 g/ha bewirkten die Verbindungen gemäß den Beispielen 84 und 88 nach 15 Tagen eine Abtötung von 98 bzw. 99 %.

Beispiel L1

**Brevicoryne brassicae - Test; Freiland** (Drenchapplikation)

Zur Herstellung einer zweckmäßigen Wirkstoffzubereitung vermischt man 1 Gewichtsteil Wirkstoff mit den angegebenen Mengen Wasser auf die gewünschte Konzentration.
Kohlpflanzen *(Brassica oleracea),* die von allen Stadien der Mehligen Kohlblattlaus *(Brevicoryne brassicae)* befallen sind werden mit einer Wirkstoffzubereitung der gewünschten Konzentration angegossen.
Nach der gewünschten Zeit wird die Abtötung in % bestimmt. Dabei bedeutet 100 %, dass alle Blattläuse abgetötet wurden; 0 % bedeutet, dass keine Blattlaus abgetötet wurde.
Bei einer Aufwandmenge von 0,04 g / Pflanze bewirkten die Verbindungen gemäß den Beispielen 84 bzw. 88 nach 35 Tagen eine Abtötung von 100 bzw. 97 %.

Beispiel M1

**Brevicoryne brassicae - Test; Freiland** (Spritzbehandlung)

Zur Herstellung einer zweckmäßigen Wirkstoffzubereitung vermischt man 1 Gewichtsteil Wirkstoff mit den angegebenen Mengen Wasser auf die gewünschte Konzentration.
Kohlpflanzen *(Brassica oleracea),* die von allen Stadien der Mehligen Kohlblattlaus *(Brevicoryne brassicae)* befallen sind werden mit einer Wirkstoffzubereitung der gewünschten Konzentration gespritzt.
Nach der gewünschten Zeit wird die Abtötung in % bestimmt. Dabei bedeutet 100 %, dass alle Nymphen abgetötet wurden; 0 % bedeutet, dass keine Nymphe abgetötet wurde.
Bei einer Aufwandmenge von 300 g/ha bewirkten die Verbindungen gemäß den Beispielen 84 bzw. 88 nach 28 Tagen eine Abtötung von jeweils 96 bzw. 99 %.

## Patentansprüche

**1.** Verwendung von Verbindungen der Formel (I)

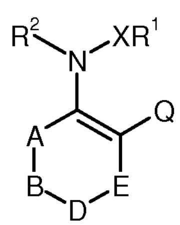

(I)

in welcher

$R^1$ für einen unsubstituierten oder substituierten 5- oder 6-gliedrigen aromatischen heterocyclischen Rest steht, der Stickstoff enthält,
X für jeweils unsubstituiertes oder substituiertes Alkylen oder Alkyliden steht,
$R^2$ für Wasserstoff, jeweils unsubstituiertes oder substituiertes Alkyl, Alkenyl, Cycloalkyl, Cycloalkenyl, Aryl oder für $YR^3$ steht,
Y für Sauerstoff, $S(O)_1$, CO oder $CO_2$ steht, 1 für 0, 1 oder 2 steht,
$R^3$ für Wasserstoff oder jeweils unsubstituiertes oder substituiertes Alkyl, Alkenyl, Alkinyl, Cycloalkyl, Cycloalkenyl oder Aryl steht,
A, B und D, unabhängig voneinander jeweils für ein unsubstituiertes oder substituiertes Kohlenstoffatom oder

Heteroatom oder für eine Einfachbindung stehen,
E für CO oder CS steht,
Q für Wasserstoff oder für jeweils unsubstituiertes oder substituiertes Alkyl, Alkenyl, Alkinyl oder Aryl oder für Nitro, Halogen oder für Z-$R^4$ steht,
Z für CO, $CO_2$ oder $S(O)_m$ steht,
m für 0, 1 oder 2 steht und
$R^4$ für jeweils unsubstituiertes oder substituiertes Alkyl, Alkenyl, Alkinyl, Cycloalkyl, Cycloalkenyl oder Aryl steht

zur Behandlung von Saatgut.

**2.** Saatgut, behandelt mit mindestens einer Verbindung der Formel (I).

**3.** Verfahren zur Bekämpfung von tierischen Pflanzenschädlingen, **dadurch gekennzeichnet, dass** Verbindungen der Formel (I) gemäß Anspruch 1 auf den Boden um die Pflanzen ausgebracht werden.

**4.** Verwendung von Verbindungen der Formel (I) zur Bekämpfung tierischer Schädlinge.

**5.** Verwendung von Verbindungen der Formel (I) zur Bekämpfung der Stubenfliege.

**6.** Verwendung von Verbindungen der Formel (I) zur Bekämpfung von Schaben.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- EP 0539588 A1
- WO 02085870 A
- US 4272417 A
- US 4245432 A
- US 4808430 A
- US 5876739 A
- US 20030176428 A1
- WO 2002080675 A1
- WO 2002028186 A2
- EP 0539588 A